# EUROPEAN PATENT APPLICATION

(11) **EP 2 532 751 A1**
(43) Date of publication of application: **12.12.2012**
(21) Application number: 11305730.1
(22) Date of filing: 10.06.2011
(51) Int. Cl.: C12P 7/18, C12N 9/02

(54) **Use of inducible promoters in the fermentative production of 1,2-propanediol**

(71) Applicant: Metabolic Explorer, 63360 Saint Beauzire (FR)
(72) Inventor: Renaud, Stéphanie, 63360 Gerzat (FR); Voelker, François, 42210 Montrond les Bains (FR); Figge, Rainer, 63450 Le Crest (FR)
(74) Representative: Regimbeau

(57) **Abstract**

The present application is related to a method for the production of 1,2-propanediol in a fermentative process comprising the following steps:

- culturing a modified microorganism in an appropriate culture medium comprising a source of carbon,
- modulating in said microorganism the level of expression of the gene *gapA* via an external stimulus and
- recovering 1,2-propanediol from the culture medium,
wherein in said modified microorganism, the expression of the gene *gapA* coding for the enzyme GAPDH is under the control of a heterologous inducible promoter. Advantageously, the inducible promoter is regulated by temperature, via a modified lambda repressor allele. The application is also related to a genetically modified microorganism for an improved production of 1,2-propanediol.

## Description

### FIELD OF THE INVENTION

The present invention relates to use of inducible promoters in the production of 1,2-propanediol by fermentation. The use of inducible promoters leads to a more stable 1,2-propanediol producer strain and higher productivity.

### BACKGROUND OF THE INVENTION

The present invention concerns a method for the production of 1,2-propanediol, with a metabolically engineered micro-organism producing 1,2-propanediol, whose gene *gapA* is under the control of an inducible promoter.

1,2-propanediol or propylene glycol, a C3 di-alcohol with formula C3H8O2 or HO-CH2-CHOH-CH3, is a widely-used chemical. Its CAS number is 57-55-6. It is a colorless, nearly odorless, clear, viscous liquid with a faintly sweet taste, hygroscopic and miscible with water, acetone, and chloroform. It is a component of unsaturated polyester resins, liquid detergents, coolants, anti-freeze and de-icing fluids for aircrafts. Propylene glycol has been increasingly used since 1993-1994 as a replacement for ethylene derivatives, which are recognised as being more toxic than propylene derivatives.

1,2-propanediol is currently produced by chemical means using a propylene oxide hydration process that consumes large amounts of water, uses highly toxic substances and generates by-products such as *tert*-butanol and 1-phenyl ethanol.

The disadvantages of the chemical processes for the production of 1,2-propanediol make biological synthesis an attractive alternative. Two routes have been characterized for the fermentative production of 1,2-propanediol from sugars by microorganisms.

In the first route, 6-deoxy sugars (*e.g*. L-rhamnose or L-fucose) are cleaved into dihydroxyacetone phosphate and (S)-lactaldehyde, which can be further reduced to (S)-1,2-propanediol (Badia *et al.,* 1985). This route is functional in *E. coli,* but can not yield an economically feasible process due to the elevated cost of the deoxyhexoses.

The second route is the metabolism of common sugars (*e.g*. glucose or xylose) through the glycolysis pathway followed by the methylglyoxal pathway. Dihydroxyacetone phosphate is converted to methylglyoxal that can be reduced either to lactaldehyde or to acetol. These two compounds are then transformed into 1,2-propanediol. This route is used by natural producers of (R)-1,2-propanediol, such as *Clostridium sphenoides* and *Thermoanaerobacter thermosaccharolyticum.* However, improvement of the performances obtained with these organisms is likely to be limited due to the lack of available genetic tools.

### PRIOR ART

Several investigations for genetic modifications *of E. coli* in order to obtain a 1,2-propanediol producer using simple carbon sources have been led by the groups of Cameron (Cameron *et al.,* 1998; Altaras and Cameron, 1999; Altaras and Cameron, 2000) and Bennett (Huang *et al.,* 1999, Berrios-Rivera *et al.,* 2003). These studies rely, on the one hand, on the expression of one or several enzymatic activities in the pathway from dihydroxyacetone phosphate to 1,2-propanediol, and on the other hand, on the removal of NADH and carbon consuming pathways in the host strain.

The best results obtained by the group of Cameron are production of 1.4 g/L 1,2-propanediol in anaerobic flask culture with a yield of 0.2 g/g of glucose consumed. If extrapolated to anaerobic fed-batch fermenter, the production would be 4.5 g/L 1,2-propanediol with a yield of 0.19 g/g from glucose. These performances have been obtained with the overexpression of the methylglyoxal synthase gene of *E. coli* (*mgs*), the glycerol dehydrogenase gene of *E. coli* (*g*/*dA*) and the 1,2-propanediol oxidoreductase gene of *E. coli* (*fuc*O) in a strain lacking the gene coding for lactate dehydrogenase *(ldhA).* Results obtained with the same approach but with lower titers and yields are also described in the patents US 6,087,140, US 6,303,352 and WO 98/37204.

The group of Bennett also used an *E. coli* host strain lacking *ldh*A for the overexpression of the *mgs* gene from *Clostridium acetobutylicum* and the *gld*A gene from *E. coli.* Flask cultures under anaerobic conditions gave a titer of 1.3 g/L and a yield of 0.12 g/g whereas microaerobic cultures gave a titer of 1.4 g/L with a yield of 0.13 g/g.

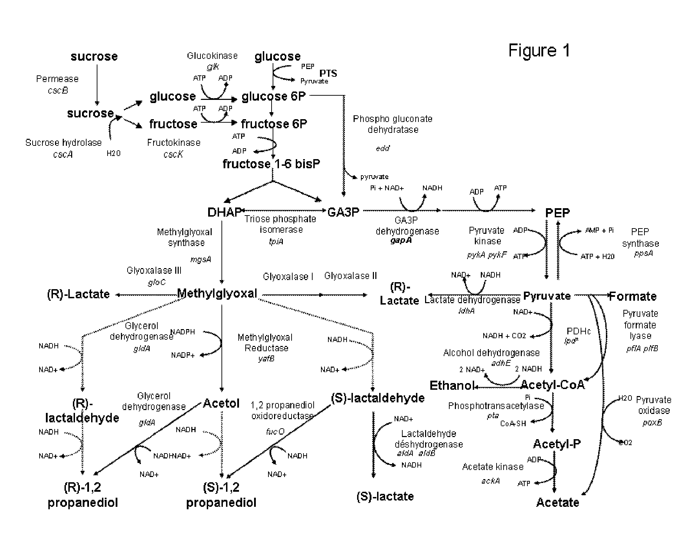

The patent application WO 2008/116848 describes a microorganism with an attenuated activity of the GAPDH enzyme, showing an improved production of 1,2-propanediol. The glyceraldehyde 3 phosphate activity is attenuated in order to redirect a part of the available glyceraldehyde 3 phosphate towards the synthesis of 1,2-propanediol via the action of the enzyme triose phosphate isomerase (see figure 1). In these conditions, the yield of 1,2-propanediol over glucose is improved. This patent application is hereby included by reference.

### BRIEF DESCRIPTION OF THE INVENTION

Efficient 1,2-propanediol production requires fine tuning of pathways. For maximum 1,2-propanediol production and improved stability of producer strains, it is beneficial to modulate the expression of certain key enzymes during the process.

As previously disclosed, attenuation of the expression of the gene *gapA* is beneficial to improve the 1,2-propanediol production yield over glucose; however, this modification can also lead to a decreased general metabolism of the microorganism. Therefore, it is preferable to attenuate said gene only when necessary.

The inventors have found that heterologous inducible promoters can be used to regulate specifically genes expression in complex metabolic pathways such as 1,2-propanediol biosynthesis, in particular to modulate the expression level of the gene *gapA* on demand, via an external stimulus. In particular, in the modified microorganism producing 1,2-propanediol, the gene *gapA* can be under the direct control of an inducible promoter.

The present invention concerns a method for the production of 1,2-propanediol in a fermentative process comprising the following steps:
- culturing a modified microorganism in an appropriate culture medium comprising a source of carbon,
- modulating in said microorganism the level of expression of the gene *gapA* via an external stimulus, and
- recovering 1,2-propanediol from the culture medium,
wherein in said modified microorganism, the expression of the gene *gapA* coding for the enzyme GAPDH is under the control of a heterologous inducible promoter.

The invention also concerns the microorganism modified for 1,2-propanediol production in which expression of the gene *gapA* is under the control of a heterologous inducible promoter. The expression of this gene can be modulated via en external stimulus, in particular by modifying the temperature of the culture medium, to optimize the production of 1,2-propanediol from glucose or sucrose. Advantageously, the inducible promoter is regulated by a specific repressor that can be mutated to optimize its action.

### BRIEF DESCRIPTION OF THE DRAWINGS

The accompanying drawing that is incorporated in and constitutes a part of this specification exemplifies the invention and together with the description, serves to explain the principles of this invention.

**Figure 1** depicts the central metabolism of the 1,2-propanediol fermentative production from carbohydrates.

### DETAILLED DESCRIPTION OF THE INVENTION

Before describing the present invention in detail, it is to be understood that this invention is not limited to particularly exemplified methods and may, of course, vary. It is also to be understood that the terminology used herein is for the purpose of describing particular embodiments of the invention only, and is not intended to be limiting, which will be limited only by the appended claims.

All publications, patents and patent applications cited herein, whether *supra* or *infra*, are hereby incorporated by reference in their entirety. However, publications mentioned herein are cited for the purpose of describing and disclosing the protocols, reagents and vectors which are reported in the publications and which might be used in connection with the invention. Nothing herein is to be construed as an admission that the invention is not entitled to antedate such disclosure by virtue of prior invention.

Furthermore, the practice of the present invention employs, unless otherwise indicated, conventional microbiological and molecular biological techniques within the skill of the art. Such techniques are well known to the skilled worker, and are explained fully in the literature. See, for example, Prescott et al. "Microbiology" (1999) 4th Edition, WCB McGraw-Hill; and Sambrook et al., "Molecular Cloning: A Laboratory Manual" (1989) (2001), 2nd & 3rd Editions, Cold Spring Harbor Laboratory Press.

It must be noted that as used herein and in the appended claims, the singular forms "a", "an", and "the" include plural reference unless the context clearly dictates otherwise. Thus, for example, a reference to "a microorganism" includes a plurality of such microorganisms, and a reference to "an endogenous gene" is a reference to one or more endogenous genes, and so forth. Unless defined otherwise, all technical and scientific terms used herein have the same meanings as commonly understood by one of ordinary skill in the art to which this invention belongs. Although any materials and methods similar or equivalent to those described herein can be used to practice or test the present invention, the preferred materials and methods are now described.

As used herein, the following terms may be used for interpretation of the claims and specification.

In the claims which follow and in the preceding description of the invention, except where the context requires otherwise due to express language or necessary implication, the word "comprise" or variations such as "comprises" or "comprising" is used in an inclusive sense, i.e. to specify the presence of the stated features but not to preclude the presence or addition of further features in various embodiments of the invention.

The present invention is related to a method for the production of 1,2-propanediol in a fermentative process comprising the following steps:
- culturing a modified microorganism in an appropriate culture medium comprising a source of carbon,
- modulating in said microorganism the level of expression of the gene *gapA* via an external stimulus and
- recovering 1,2-propanediol from the culture medium,
wherein in said modified microorganism, the expression of the gene *gapA* coding for the enzyme GAPDH is under the control of a heterologous inducible promoter.

According to the invention, the terms "fermentative process', 'fermentation" or 'culture' are used interchangeably to denote the growth of bacteria in an appropriate growth medium.

An "appropriate culture medium" is a medium appropriate for the culture and growth of the microorganism. Such media are well known in the art of fermentation of microorganisms, depending upon the microorganism to be cultured. As an example of a known culture medium for *E. coli,* the culture medium can be of identical or similar composition to M9 medium (Anderson, 1946), M63 medium (Miller, 1992; A Short Course in Bacterial Genetics: A Laboratory Manual and Handbook for Escherichia coli and Related Bacteria, Cold Spring Harbor Laboratory Press, Cold Spring Harbor, New York) or a medium such as defined by Schaefer *et al.* (1999), and in particular the minimum culture medium named MPG described below:

| | |
|---|---|
| **K₂HPO₄** | 1.4 g/l |
| **Nitrilo Triacetic Acid** | 0.2 g/l |
| **trace element solution*** | 10 ml/l |
| **(NH₄)₂SO₄** | 1 g/l |
| **NaCl** | 0.2 g/l |
| **NaHCO₃** | 0.2 g/l |
| **MgSO₄** | 0.2 g/l |
| **glucose** | 20 to 100 g/l |
| **NaNO₃** | 0.424 g/l |
| **thiamine** | 10 mg/l |
| **FeSO₄, 7H₂O** | 50 mg/l |
| **yeast extract** | 4 g/l |

The pH of the medium is adjusted to 7.4 with sodium hydroxide.

****trace element solution** :* Citric acid 4.37 g/L, MnSO₄ 3 g/L, CaCl₂ 1 g/L, CoCl₂, 2H₂O 0.1 g/L, ZnSO₄, 7H₂O 0.10 g/L, CuSO₄, 5H₂O 10 mg/L, H₃BO₃ 10 mg/L, Na₂MoO₄ 8.31 mg/L.

The term "source of carbon" refers to any carbon source capable of being metabolized by a microorganism wherein the substrate contains at least one carbon atom.

The source of carbon is selected among the group consisting of glucose, sucrose, monosaccharides (such as fructose, mannose, xylose, arabinose), oligosaccharides (such as galactose, cellobiose ...), polysaccharides (such as cellulose), starch or its derivatives, glycerol and single-carbon substrates whereby glyoxylic acid is produced. An especially preferred carbon source is glucose. Another preferred carbon source is sucrose.

In a particular embodiment of the invention, the carbon source is derived from renewable feed-stock. Renewable feed-stock is defined as raw material required for certain industrial processes that can be regenerated within a brief delay and in sufficient amount to permit its transformation into the desired product.

In a specific embodiment of the invention, the method is performed with a strain of *E. coli* grown in a medium containing a simple carbon source that can be arabinose, fructose, galactose, glucose, lactose, maltose, sucrose or xylose. An especially preferred simple carbon source is sucrose.

The fermentation is generally conducted in fermenters with an appropriate culture medium adapted to the microorganism being used, containing at least one simple carbon source. Those skilled in the art are able to define the culture conditions for the microorganisms according to the invention. In particular the bacteria are fermented at a temperature between 20°C and 55°C, preferentially between 25°C and 40°C, and more specifically about 30°C to 37°C for *E. coli.*

This process can be carried out either in a batch process, in a fed-batch process or in a continuous process. It can be carried out under aerobic, micro-aerobic or anaerobic conditions.

'Under aerobic conditions' means that oxygen is provided to the culture by dissolving the gas into the liquid phase. This could be obtained by (1) sparging oxygen containing gas (e.g. air) into the liquid phase or (2) shaking the vessel containing the culture medium in order to transfer the oxygen contained in the head space into the liquid phase. The main advantage of the fermentation under aerobic conditions is that the presence of oxygen as an electron acceptor improves the capacity of the strain to produce more energy under the form of ATP for cellular processes. Therefore the strain has its general metabolism improved.

Micro-aerobic conditions are defined as culture conditions wherein low percentages of oxygen (e.g. using a mixture of gas containing between 0.1 and 10% of oxygen, completed to 100% with nitrogen), is dissolved into the liquid phase.

Anaerobic conditions are defined as culture conditions wherein no oxygen is provided to the culture medium. Strictly anaerobic conditions are obtained by sparging an inert gas like nitrogen into the culture medium to remove traces of other gas. Nitrate can be used as an electron acceptor to improve ATP production by the strain and improve its metabolism.

The phrase "recovering 1,2-propanediol from the culture medium" designates the process of purifying the produced 1,2-propanediol, using methods known by the man skilled in the art. Such methods are disclosed especially in PCT/EP2010/070102 and PCT/EP2011/055034 patent applications.

It will be understood that any microorganism known in the art may be useful in the present invention. In particular, the microorganism may be a bacterium, yeast or fungus. In one embodiment, the microorganism is a bacterium selected among gram positive bacteria or gram negative bacteria. Preferentially, the bacterium is selected among gram negative bacteria such as *Enterobacteriaceae,* or among gram positive bacteria such as *Bacillaceae, Streptomycetaceae* and *Corynebacteriaceae.* More preferentially, the bacterium is a species of *Escherichia, Klebsiella, Pantoea, Salmonella* or *Corynebacterium.* Even more preferentially, the bacterium is either the species *Escherichia coli* or *Corynebacterium glutamicum.*

The term "modified microorganism", as used herein, refers to a microorganism that is not found in nature and is genetically different from equivalent microorganisms found in nature. The microorganism may be modified through either the introduction or deletion of genetic elements, or through a step of evolution as described in patent application WO 2005/073364. In particular, it designates a genetically modified microorganism presenting an improved 1,2-propanediol production. Such microorganism is for example described in the patent application WO 2008/116848, incorporated by reference. Preferred genetic modifications are the following:
- increased expression of at least one gene selected among *mgs*A, *yqh*D, *yaf*B, *ycd*W, *yqh*E, *yea*E, *ygh*Z, *yaj*O, *tas, ydj*G, *ydb*C, *gld*A *and fuc*O;
- deletion of either the *edd* or *eda* gene or both;
- attenuation of the synthesis of unwanted by-products by deletion of the genes coding for enzymes involved in synthesis of lactate from methylglyoxal (such as *glo*A, *ald*A, *ald*B)*,* lactate from pyruvate (*ldh*A), formate (*pfl*A, *pfl*B), ethanol (*adh*E) and acetate (*ack*A, *pta, pox*B);
- elimination of the pathways consuming PEP like pyruvates kinases (encoded by the *pyk*A and *pyk*F genes) and/or by promoting the synthesis of PEP *e.g*. by overexpressing the *pps*A gene coding for PEP synthase;
- specific mutation in the *lpd* gene;
- the *arc*A and the *ndh* genes can be deleted,
- genes involved in the importation and metabolism of sucrose (*cscB*, *cscA*, *cscK*) are added or their expression is increased.

A preferred genetic modification is the improvement of methylglyoxal reductase activity, obtained by an increased expression of the gene *yafB.*

Another preferred genetic modification is the improvement of methylglyoxal synthase activity, obtained by an increased expression of the gene *mgsA* (H21 Q).*

According to the invention, in said modified microorganism, the level of expression of the gene *gapA* encoding the enzyme GAPDH can be modulated via an external stimulus. This is advantageous, since a normal expression of the enzyme GAPDH is important for the normal growth of the cells, but an attenuated expression is favourable for the production of 1,2-porpanediol : by modifying the external stimulus, the culture of the cells can be optimized alternatively for the growth of the cells or for the production of 1,2-propanediol.

The glyceraldehyde 3-phosphate dehydrogenase, also called GAPDH, is one of the key enzymes involved in the glycolytic conversion of glucose to pyruvic acid. GAPDH catalyzes the following reaction:

Glyceraldehyde 3-phosphate + phosphate + NAD⁺ → 1,3-biphosphoglycerate + NADH + H⁺

The gene encoding this enzyme was cloned in 1983 in *E*. *coli* (Branlant *et al.,* 1983) and named *"gapA".*

The term "modulation" refers to increasing or decreasing the level of expression of the gene *gapA.*

The term "expression" refers to the transcription and translation of a gene sequence leading to the generation of the corresponding protein, product of the gene.

It is understood that the "normal" level of expression will be considered at 100%; a "decreased" expression will be less than 80%; and an "increased" expression will be up to 120%.

The modulation of the level of expression of the gene *gapA* will be performed via an external stimulus, acting on an heterologous inducible promoter.

The term "inducible promoter" denotes a promoter whose activity can be increased or decreased upon an external stimulus. These promoters usually respond to chemical or physical stimuli such as propionate (WO2007005837), zinc (WO2004020640), arabinose (WO1998011231), light or temperature (Tang *et al.,* 2009).

Induction of the target gene can be obtained via direct or indirect transmission of the stimulus.

Direct transmission is accomplished when the expression of one target gene is under the control of an inducible promoter.

Indirect transmission can be accomplished by using heterologous RNA-polymerases that are under the control of an inducible promoter and that recognize specific promoters driving the expression of target genes involved in 1,2-propanediol biosynthesis. In this case, the inducible promoter is not directly linked to the promoter of the target gene, but drives the expression of an RNA polymerase transcribing said promoter of the target gene.

These heterologous RNA polymerases can be e.g. T3 RNA polymerase, T7 RNA polymerase or other polymerase known to the expert in the field.

'Indirect transmission' also refers to the 'polar effect' of the inducible expression of one specific gene on the expression of its neighbouring genes. A "polar effect" designates the influence of a genetic modification in a gene on the expression of one or more genes which are downstream said modified gene.

The phrase "under the control of a heterologous inducible promoter" designates the fact that the inducible promoter is not the native promoter of the gene and was introduced in a way to control, at least partially, the level of expression of the gene that is operably linked to it. The activity of an inducible promoter is induced by external stimuli.

Introduction of an heterologous promoter in front of the endogenous *gapA* gene is a routine task for the man skilled in the art: see in particular the examples.

In a first aspect of the invention, the external stimulus is a physical stimulus, in particular linked to the temperature or to the light. In a preferred embodiment of the invention, the stimulus is the temperature of the culture medium.

The promoter is advantageously a temperature-inducible promoter from the bacteriophage lambda, chosen among: the promoter PR or a derivative of PR, the promoter PL or a derivative of PL, or a modified lac promoter regulated by a temperature sensitive Lac repressor.

In a preferred embodiment of the invention, the gene *gapA* is under the control of the promoter PR from the bacteriophage lambda.

The bacteriophage lambda (Enterobacteriophage λ, lambda phage, coliphage λ) is a temperate bacteriophage that infects *E.coli.* The infected bacterium is maintained at the lysogenic state by the action of the 'lambda repressor', that represses the expression of the phage genes. When the lysogen is induced by UV light for example, the repressor is cleaved and the lysis of the bacteria is induced.

The 'lambda repressor' represses the expression from PR or PL promoter by binding to specific binding sites in the promoter region, thereby limiting the access of RNA polymerase to the promoter and reducing initiation or elongation of transcription.

The repressor CI has been isolated (Ptashne, 1967) and extensively described in the art; see in particular: Roberts JW & Roberts CW, 1975,Pabo CO *et al.,* 1979, andSussman et al., 1962.

This thermoregulated system has been shown to function in a range of species, induction of genes being achieved by elevation of the temperature from 28 to 37°C (Mandal & Lieb, 1976, and Winstanley *et al.,* 1989).

The CI lambda repressor is a protein of 236 amino acids that binds to operator sites on the DNA, blocking transcription of genes under the control of the promoters with CI binding sites. Under physiological conditions, in the lytic state of the bacteriophage lambda, the repressor CI is proteolytically inactivated, and then the genes previously inhibited are expressed. Proteolytic cleavage of CI is mediated by its binding to a helical filament of the host RecA protein formed at a site of DNA damage, in particular following an elevation of the temperature. Though initially RecA was thought to be a protease that cleaves CI, it eventually became clear that the repressor actually undergoes an intramolecular self-cleavage at the peptide bond between Ala111 and Gly112, but only when bound to a RecA-ssDNA-ATP filament. The structure of hyper-cleavable forms of the repressor have been studied to understand better the mechanism of the self-cleavage (Ndjonka & Bell, *2006*).

In a preferred embodiment of the invention, the CI lambda repressor is the lambda repressor allele CI857, described in Sussman et al. (1962), or another temperature-sensitive allele of the CI lambda repressor. Sussman et al. report a new mutant of the bacteriophage, being in the lysogenic state when cultivated at 32°C, but wherein its lyse is induced when the culture is maintained at a temperature of 40°C for one hour.

To optimize the system of repression of the expression of the gene *gapA,* placed under the control of a promoter PR or PL, it is advantageous that the CI repressor be resistant to cleavage. Different mutations of the CI repressor have been reported, that improve the stability and resistance to cleavage of the CI repressor: see in particular (Gimble & Sauer, 1985) and (Gimble & Sauer, 1986) that reports the behaviour of 16 mutants of lambda repressors.

In a specific embodiment of the invention, the used repressor allele CI is a mutant form resistant to cleavage.

Presence of the mutation E117K in CI857 repressor increases the efficiency of the self-cleavage, compared to the wild-type protein; but cleavage by the protein RecA is abolished. In a specific aspect of the invention, this mutation is suppressed in the used CI857 repressor, the wild type amino acid (E) being restored in this CI857 repressor.

In a particular embodiment of the invention, the cleavage site placed at the peptide bond between Alanine 111 and Glycine 112 of the repressor allele CI is deleted or mutated in the used mutant form of CI. In particular the following mutations: A111T or G112E suppress the cleavage site and A111G or G112A modify the cleavage site but do not suppress it totally.

The following mutations or combinations thereof limit the cleavage mediated by the RecA protein, but have no effect on the self-cleavage: G124D, D125V, D125Y, G185N, G185E, F189L, E127K, T122I and D125N.

A particularly relevant mutation is the point mutation G147D that suppresses both self-cleavage and RecA mediated cleavage of the mutant CI protein.

In a preferred embodiment of the invention, the mutant form of the repressor allele CI857 comprises at least one of these mutations: G147D, G124D, D125N, D125V, D125Y, G185N, G185E, F189L, E127K, and T122I.

According to a specific embodiment of the invention, the *recA* gene in the modified microorganism has been deleted. In this specific embodiment of the invention, the cleavage of the CI repressor is abolished.

A Ribosome Binding Site (RBS) is, in the mRNA, the region wherein the ribosome will fix in order to translate the mRNA into protein. A Ribosome Binding Site (RBS) is a sequence on mRNA that is bound by the ribosome when initiating protein translation. It can be either the 5' cap of a messenger RNA in eukaryotes, a region 6-7 nucleotides upstream of the start codon AUG in prokaryotes (called the Shine-Dalgarno sequence), or an internal ribosome entry site (IRES) in viruses. The sequence is complementary to the 3' end of the rRNA. The ribosome searches for this site and binds to it through base-pairing of nucleotides. Then, the ribosome begins the translation process and recruits initiation factors. After finding the ribosome binding site in eukaryotes, the ribosome recognizes the Kozak consensus sequence and begins translation at the +1 AUG codon.
One way to attenuate the expression of a gene is the modification of the wild type RBS. In particular for the *gapA* gene, the replacement of the wild type RBS allows the attenuation of the expression of the gene.
In a particular embodiment of the invention, the wild type RBS upstream of the codon AUG of the mRNA from the *gapA* gene is replaced with RBS01 *2 or RBS11.
The wild type sequence of the RBS is the following: ***RBSgapA,*** TAGC**TGGTGG**AATAT (from 1860780 to 1860794 on *E*. *coli* chromosome) (SEQ ID NO 1).
According to a specific embodiment of the invention, the mutated Ribosome Binding Site presents one of the nucleotidic sequences:
- SEQ ID NO 2 (RBS01*2) → TAAGG**AGGTTATAA,** or
- SEQ ID NO 3 (RBS11) → TA**AGGAGA***TTATAA*

The method according to the invention allows the modulation of the level of expression of the *gapA* gene during the culture of the microorganism producing 1,2-propanediol ; in decreasing the temperature of the culture medium at 30°C, the expression of the *gapA* gene is decreased, although at a temperature between 37°C to 42°C, the expression of the *gapA* gene is maintained at a normal level.

In a preferred embodiment of the invention, the modified microorganism is furthermore genetically modified for improving the production of 1,2-propanediol.

The invention is also related to a microorganism modified for an improved production of 1,2-propanediol, wherein in said modified microorganism the expression of the gene *gapA* is under the control of a heterologous inducible promoter as defined previously.

In a particular embodiment of the invention, in said microorganism, the *gapA* gene is expressed at 37°C to 42°C and has a decreased expression at 30°C.

The *gapA* gene under the control of the inducible promoter is preferentially the endogenous *gapA* gene; in another embodiment, an exogenous *gapA* gene can be integrated into loci, whose modification does not have a negative impact on 1,2-propanediol production. Examples for loci into which the gene may be integrated are: arsRBC, glcV, malS, melB, pga, purU, treBC, uxaCA, wcaM, ybeH, ycgH, yghX, yjbI, yjjN, ykiA, yncA, yneL, ytfA, yrhC.

In the description of the present invention, genes and proteins are identified using the denominations of the corresponding genes in *E*. *coli.* However, and unless specified otherwise, use of these denominations has a more general meaning according to the invention and covers all the corresponding genes and proteins in other organisms, more particularly microorganisms.

Using the references given in GenBank for known genes, those skilled in the art are able to determine the equivalent genes in other organisms, bacterial strains, yeasts, fungi, mammals, plants, etc. This routine work is advantageously done using consensus sequences that can be determined by carrying out sequence alignments with genes derived from other microorganisms, and designing degenerate probes to clone the corresponding gene in another organism. These routine methods of molecular biology are well known to those skilled in the art, and are claimed, for example, in Sambrook *et al.* (1989 Molecular Cloning: a Laboratory Manual. 2nd ed. Cold Spring Harbor Lab., Cold Spring Harbor, New York.)

PFAM (protein families database of alignments and hidden Markov models; http://www.sanger.ac.uk/Software/Pfam/) represents a large collection of protein sequence alignments. Each PFAM makes it possible to visualize multiple alignments, see protein domains, evaluate distribution among organisms, gain access to other databases, and visualize known protein structures.

COGs (clusters of orthologous groups of proteins; http://www.ncbi.nlm.nih.gov/COG/ are obtained by comparing protein sequences from fully sequenced genomes representing major phylogenic lines. Each COG is defined from at least three lines, which permits the identification of former conserved domains.

The means of identifying homologous sequences and their percentage homologies are well known to those skilled in the art, and include in particular the BLAST programs, which can be used from the website http://www.ncbi.nlm.nih.gov/BLAST/ with the default parameters indicated on that website. The sequences obtained can then be exploited (e.g., aligned) using, for example, the programs CLUSTALW (http://www.ebi.ac.uk/clustalw/) or MULTALIN (http://bioinfo.genotoul.fr/multalin/multalin.html), with the default parameters indicated on those websites.

### EXAMPLES

### Example 1: Construction and evaluation of strains with gapA controlled by a thermoregulated promoter in MG1655

### 1. Construction of strain MG1655 CI857-PR01/RBS01*2-gapA::Km

The substitution of natural *gapA* promoters by a thermoregulated one was done by the simultaneous insertion of a resistance antibiotic cassette and the new promoter according to Protocol 1. This method was also used to delete genes.

Protocol 1: Introduction of a PCR product for recombination and selection of the recombinants (FRT system).

The oligonucleotides chosen and given in Table 1 for replacement of a gene or an intergenic region were used to amplify either the chloramphenicol resistance cassette from the plasmid pKD3 or the kanamycin resistance cassette from the plasmid pKD4 (Datsenko & Wanner, 2000). The PCR product obtained was then introduced by electroporation into the recipient strain bearing the plasmid pKD46 in which the expressed λ Red (γ, β, exo) system greatly favours homologous recombination. The antibiotic-resistant transformants were then selected and the insertion of the resistance cassette was checked by PCR analysis with the appropriate oligonucleotides given in Table 2.

If there were others modifications in the strain, these were checked with the oligonucleotides given in Table 2.

The PCR fragment used here was obtained by overlapping PCR. The first fragment contains TTadc-*CI857*-PR01 and the second one the kanamycin resistance cassette. Firstly, the fragment corresponding to the TTadc-*CI857*-PR01 was PCR amplified from the pFC1 vector (Mermet-Bouvier & Chauvat, 1994), using the following oligonucleotides : TTadc-CI857-F-1 and PR01/RBS01*2-gapA.

Secondly, the fragment corresponding to the kanamycin resistance cassette (Km) was PCR amplified from pKD4 vector using the following oligonucleotides: Ptrcgap F and Km/frt R.

Thirdly, the region Km-TTadc-CI857-PR01 with homology to the upstream region of *gapA* was PCR amplified by mixing the TTadc-CI857-PR01/RBS01*2 and Km products, which possess a 30 bp overlapping sequence, using PR01/RBS01*2-gapA and Ptrcgap F. Then the PCR product was used according to Protocol 1.
The resulting strain was named *E*. *coli* MG1655 CI857-PR01/RBS01*2-*gapA.*

**Table 1: Oligonucleotides used for replacement or insertion of a chromosomal region by recombination with a PCR product**

| **Region name** | **Names of oligos** | **SEQ ID NO** | **Sequence** | **Details about oligonuleotides Homology with chromosomal region (http://ecogene.org/blast.php)** |
|---|---|---|---|---|
| *gapA* | •TTadc-CI857-F-1 | 4 | •tcagccaaacgtctcttc aggccactgactag | • region homologous to pFCl. |
| | •PR01/RBS 01*2-gapA | 5 | •atgtcagaacgtttctga gcagcacggaaaacaat gcgaccgatacggccaa aaccgttgatacctactttg atagtcatTTATAAC CTCCTTAgtacatg caaccattatcacc | • - Sequence homologous pFCl (Italic letters) - region for the addition of RBS01*2 ribosome binding site region (capital letters) -region homologous to E. coli chromosome (small letters) |
| | •PR01/RBS 11-gapA | 6 | •atgtcagaacgtttctga gcagcacggaaaacaat gcgaccgatacggccaa aaccgttgatacctactttg atagtcatTTATAAT CTCCTTAgtacatg caaccattatcac | • - Sequence homologous pFCl (Italic letters) - region for the addition of RBS01*2 ribosome binding site region (capital letters) -region homologous to E. coli chromosome (small letters) |
| | •Ptrcgap F | 7 | •ctcacatctcactttaatc gtgctcacattacgtgact gattctaacaaaacattaa caccaactggcaaaatttt gtcccatatgaatatcct ccttag | • - region homologous to E. coli chromosome (small letters) from 1860457 to 1860517 |
| | •Km/frt R | 8 | •agtcagtggcctgaag agacgtttggctgataaa aaaaataagagttaccatt taaggtaactcttatttttag ggccctgtaggctggag ctgcttcg | • - region for addition of a Terminator TTadc (underlined letters) -a region homologous to pFCl to overlap region with the first PCR fragment (Italic letters) |
| CI857 | •CI857 gapA fusion F | 9 | •ctcacatctcactttaatc gtgctc | • region homologous to E. coli chromosome (small letters) |
| | •CI857*( G112E) R | 10 | •ggcagAgatgttctcac ctGaActccgtaccttta ccaaaggtgatgc | • homologous region to CI857 with in bold capital letters modified bases to remove the mutation E117K and with underlined capital letter modified base to create mutation G112E. |
| | •CI857*( G113E/E1 17E) F | 11 | •gcatcacctttggtaaag gtacggagTtCaggtg agaacatcTctgcc | • homologous region of CI857 with in bold capital letters modified bases to remove the mutation E117K and with underlined capital letter modified base to create mutation G112E. |
| | •CI857 gapA fusion 5 | 12 | •atgtcagaacgtttctga gcagc | • region homologous to E. coli chromosome from 10860874 to 1860582 |
| *edd-eda* | DedaR | 13 | •gcttagcgccttctacag cttcacgcgccagcttagt aatgcggtcgtaatcgcc cgcttccagcgcatctgc cggaacccatatgaata tcctccttag | •region homologous to *E*. *coli* chromosome from 1930144 to 1930223 |
| | DeddF | 14 | •cgcgcgagactcgctct gcttatctcgcccggatag aacaagcgaaaacttcga ccgttcatcgttcgcagtt ggcatgcggtgtaggct ggagctgcttcg | •region homologous to *E*. *coli* chromosome from 1932501 to 1932582 |
| aldA | DaldAR | 15 | •ttaagactgtaaataaac cacctgggtctgcagatat tcatgcaagccatgtttac catctgcgccgccaatac cggatttcatatgaatat cctccttag | •region homologous to *E*. *coli* chromosome from 1487615 to 1487695 |
| | DaldAF | 16 | •atgtcagtacccgttcaa catcctatgtatatcgatg gacagtttgttacctggcg tggagacgcatggattga tgtggtagtgtaggctgg agctgcttcg | •region homologous to *E*. *coli* chromosome from 1486256 to 1486336 |
| aldB | DaldBR | 17 | •atgaccaataatcccccct tcagcacagattaagccc ggcgagtatggtttcccc ctcaagttaaaagcccgc tatgacaactgtaggctg gagctgcttcg | •region homologous to *E*. *coli* chromosome from 3754534 to 3754453 |
| | DaldBF | 18 | •tcagaacagccccaac ggtttatccgagtagctca ccagcaggcacttggttt gctggtaatgctccagcat catcttgtcatatgaatat cctccttag | •region homologous to *E*. *coli* chromosome from 3752996 to 3753075 |
| arcA | DarcAF | 19 | •ccccgcacattcttatcg ttgaagacgagttggtaa cacgcaacacgttgaaaa gtattttcgaagcggaag gctatgtgtaggctggag ctgcttcg | •region homologous to *E*. *coli* chromosome from4637868 to 4637791 |
| | DarcAR | 20 | •ccagatcaccgcagaa gcgataaccttcaccgtg aatggtggcgatgatttcc ggcgtatccggcgtagat tcgaaatgcatatgaata tcctccttag | •region homologous to *E*. *coli* chromosome from 4637167 to 4637245 |
| ndh | DndhF | 21 | •ctctcacaaattcgctca aataataaacaataaactc tgtttttgatctcacccggt aaagtcgcctatcttttcag ctgtaggctggagctgc ttcg | •region homologous to *E*. *coli* chromosome from1165071 to 1165149 |
| | DndhR | 22 | •gcaacttcaaacgcgga cggataacgcggttaata ctccccaccagcatcatta atccggttttaaagtaacc atgcagccatatgaatat cctccttag | •region homologous to *E*. *coli* chromosome from 1166607 to 1166528 |
| mgsA | mgsA ::K m F | 23 | •tccagtcgccgcatttca acgacgcggtcgatattct gatccccgattatcagcgt tatctcgcggaccgtctga agtaatgtaggctggag ctgcttcg | •region homologous to *E*. *coli* chromosome from 1025859 to 1025780 |
| | mgsA ::K m R | 24 | •tgtggaaatactgaaaa atctggatgtgccggtgg cgagaaaaccgtaagaa acaggtggcgtttgccac ctgtgcaatacatatgaa tatcctccttag | •region homologous to *E*. *coli* chromosome from 1025700 to 1025779 |
| gloA | DgloA F | 25 | •Atgcgtcttcttcatacc atgctgcgcgttggcgatt tgcaacgctccatcgatttt tataccaaagtgctgggc atgaatgtaggctggag ctgcttcg | •region homologous to *E*. *coli* chromosome from1725861 to 1725940 |
| | DgloA R | 26 | •Ttagttgcccagaccgc gaccggcgtctttctcttc gattaactcaattttgtaac cgtccggatcttccacaa acgcgacatatgaatat cctccttag | •region homologous to *E*. *coli* chromosome from 1726268 to 1726189 |

| | | | | |
|---|---|---|---|---|
| Sequences complementary to the Kan or Cm resistance cassette (pKD4 or pKD3) are underlined. | | | | |

**Table 2: Oligonucleotides used for checking the insertion or the loss of the resistance cassette**

| **Region name** | **Names of oligos** | **SEQ ID** | **Sequence** | **Homology with chromosomal region** |
|---|---|---|---|---|
| edd-eda genes | edaR | 27 | • ccacatgataccgggatggtgacg | • 1929754 to 1929777 |
| | eddF | 28 | • gggtagactccattactgaggcgtgggcg | • 1932996 to 1932968 |
| aldA gene | aldAF | 29 | • cgttatctctcactactcactggcg | • 1485877 to 1485901 |
| | aldAR | 30 | • cctccgcctctttcactcattaagac | • 1487714 to 1487689 |
| aldB gene | aldBF | 31 | •catatttccctcaaagaatataaaaaagaacaattaacgc | •3752449 to 3752488 |
| | aldBR | 32 | •tatgttcatgcgatggcgcaccagctgggcg | •3755070 to 3755040 |
| arcA gene | arcAF | 33 | •cgacaattggattcaccacg | •4638746 to 4638727 |
| | arcAR | 34 | •gcggtattgaaaggttggtgc | •4637308 to 4637328 |
| ndh gene | ndhF | 35 | •ccgtgagaagaatcgcgatcg | • 1164722 to 164742 |
| | ndhR | 36 | •gcgtagtcgtgtaagtatcgc | • 1167197 to 1167177 |
| gloA gene | gloAF2 | 37 | •cggtggcatttggtcgtgac | •1725641 to 1725660 |
| | gloAR2 | 38 | •gctgtttcaacatcgatcac | •1726450 to 1726431 |
| mgsA gene | yccT R | 29 | •gatggcagatgacagtacgc | •1026499 to 1026480 |
| | aval mgsA*(A95V) | 40 | •ggagtcgattatggaagaggcg | •1025559 to 1025580 |
| gapA | yeaAF | 41 | •gccacagccggaatcatacttggtttggg | •1860259 to 1860287 |
| | yeaD R | 42 | •cagcgtaaagtcgtgcggccag | •1862284 to 1862263 |

### 2. Construction of strain MG1655 CI857-PR0/RBS11-gapA::Km

This strain was constructed as described for strain MG1655 CI857-PR01/RBS01*2-*gapA,* except that one oligonucleotide was modified: PR01/RBS01*2-gapA was substituted by PR01/RBS11-gapA in order to deviate the sequence of the Ribosome Binding Site from the consensus sequence and consequently to decrease *gapA* expression.

### 3. Assessment of gapA expression in two strains with thermoregulated gapA and differing only by the Ribosome Binding Site sequence in front of gapA .

Flask cultures were carried out in order to appraise the effect of the constructions described above on growth rate and GAPDH activity. A minimal medium (MML11PG1_100, see composition in Table 3) with 10 g/L glucose as sole carbon source was used. The cultures were incubated either at 37°C or 30°C under aerobic conditions. 20 g/l MOPS were added in culture medium in order to maintain a pH above 6.0 throughout fermentation course. Growth rates are reported in Table 4.

Cells were harvested to measure glyceraldehyde-3-phosphate dehydrogenase (GAPDH) activity. Cells (25mg of dry weight) were suspended in potassium phosphate buffer and transferred into glass-bead containing tubes for lysis using Precellys (30s at 6500rpm, Bertin Technologies). Cell debris were removed by centrifugation at 12000g (4°C) during 30 minutes. Protein concentration was determined using Bradford assay.

GAPDH activity was quantified spectrophotometrically as follows. The reaction mixture contained in a 1mL final volume, 50 mM potassium phosphate, 20 mM potassium arsenate, 3 mM dithiothreitol, 0,2 mM EDTA, 1,25 mM NAD⁺, 1,25 mM glyceraldehyde-3-phosphate and 0,15 or 3 µg of cell extract. After 5 minutes of incubation at 30°C the reaction was started by adding glyceraldehyde-3-phosphate. The enzymatic activity was determined at 340 nm and 30°C by the linear rate of NADH formation. NADH formed was calculated using an extinction coefficient of 6290 M⁻¹ cm⁻¹. One unit corresponds to 1µmol of product formed by minute in the assay conditions. The activity value determined without cell extract in the assay was subtracted.

**Table 3 : Composition of minimal medium MML11PG1_100**

| **Constituent s** | **Concentration (g/L)** |
|---|---|
| Carbon source (glucose or sucrose) | 10 |
| (NH₄)2SO₄ | 4.88 |
| Citrate, H₂O (C₆H₈O₇,H₂O | 1.70 |
| KH₂PO₄ | 1.65 |
| MgSO₄, 7H₂O | 1.00 |
| K₂HPO₄, 3H₂O | 0.92 |
| (NH₄)₂HPO₄ | 0.40 |
| Fe(III) citrate, H₂O | 0.1064 |
| CaCl₂, 2H₂O | 0.08 |
| MnCl₂, 4H₂O | 0.0150 |
| Zn(CH₃COO)₂, 2H₂O | 0.0130 |
| Thiamine, HCl | 0.0100 |
| EDTA, 2Na, 2H₂O (C₁₀H₁₆N₂O₈, 2Na, 2H₂O) | 0.0084 |
| H₃BO₃ | 0.0030 |
| CoCl₂, 6H₂O | 0.0025 |
| Na₂MoO₄, 2H₂O | 0.0025 |
| CuCl₂, 2H₂O | 0.0015 |

**Table 4: Growth rate (µ) and GAPDH activity as determined during aerobic flask cultures on minimal medium**

| **Genotype** | **Temperature** | **µ (h⁻¹)** | **GAPDH (mUI/mg)** | **Ratio of GAPDH at 30°C/37°C** |
|---|---|---|---|---|
| MG1655 | **37°C** | 0.57 | 2488 | 85% |
| | **30°C** | 0.40 | 2113 | |
| MG1655 CI857-PR01/RBS01*2-*gapA::*Km | **37°C** | 0.58 | 2086 | 21% |
| | **30°C** | 0.38 | 441 | |
| MG1655 CI857-PR01/RBS11-*gapA::*Km | **37°C** | 0.50 | 593 | 21% |
| | **30°C** | 0.26 | 122 | |

The results in Table 4 show a slight decrease (-15%) of GAPDH activity in wild-type strain MG1655 at 30°C compared to 37°C, and a much higher decrease (-79%) in strains with a thermoregulated *gapA.* Indeed, at 30°C the CI857 repressor is fully functional and limits expression of the *gapA* gene. At 37°C, the CI857 repressor is denatured and allows *gapA* expression, leading to a GAPDH activity close to that of the wild-type strain with the RBS01*2 sequence, and to a much reduced activity with the RB**S11 sequence, due to a lower translation efficiency.**

### Example 2: Improvement of the stability of the thermoregulated system : construction of strain MG1655 CI857*-PR01/RBS01*2-gapA::Km

The CI857 repressor possesses 2 amino acid modifications by comparison to the wild-type CI. The first mutation, A66T, leads to the thermosensitivity of the repressor while the second mutation, E117K, avoids the cleavage of the repressor by RecA, when induced by the SOS response of the cell (Gimble & Sauer, 1986). Consequently, this CI should be more stable than the wild-type, but this mutation also induces more frequent self-cleavage of the protein (Ndjonka & Bell, 2006). The mutation E117K was thus eliminated and replaced by another one in order to avoid RecA-mediated cleavage as well as self-cleavage. The cleavage site takes place between amino acids Ala111 and Gly112. The mutation G112E abolishes both cleavage phenomenons and improves protein stability.

The construction of strain MG1655 CI857*-PR01/RBS01*2-*gapA* was done by the method described in Protocol 1. The PCR fragment used was obtained by overlapping PCR. The two fragments were PCR amplified from the genomic DNA of strain MG1655 CI857-PR01/RBS01*2-gapA::Km.

First, the fragment containing the Km cassette and the end of CI857 ORF was amplified using the following oligonucleotides : CI857 gapA fusion F and CI857*(G112E) R. Secondly, the fragment containing the beginning of the CI857 was amplified using the following oligonucleotides : CI857*(G113E/E117E) F and CI857 gapA fusion 5.

Thirdly, the final fragment with homology to the upstream region of *gapA* was PCR amplified by mixing the two PCR products, which possess a 48 bp overlapping sequence, using the oligonucleotides CI857 gapA fusion F and CI857 gapA fusion 5.

The modification was checked by a PCR analysis with the appropriate oligonucleotides given in Table 2. The resulting strain was named *E. coli* MG1655 CI857-PR01/RBS01*2-*gapA::Km.*

### Example 3 : Construction of an E. coli 1,2-propanediol producing strain with thermoregulated promoter CI857PR01/RBS01*2-gapA using glucose or sucrose as a carbon source.

### 1. Construction of strain E. coli MG1655 CI857-PR01/RBS01*2-gapA::Km mgsA ^{*}(H21Q), Δedd-eda, ΔgloA, ΔaldA, ΔaldB, ΔarcA, Δndh, pME101VB06-yqhD*(G149E)-gldA *(A160T)

### 1.1. Construction of strain E. coli MG1655 Δedd eda::Km

The *edd-eda* operon was inactivated in strain *E. coli* MG1655 by inserting a kanamycin antibiotic cassette and deleting most of the gene concerned using the technique described in Protocol 1 with the oligonucleotides given in Table 1. The deletion was checked by a PCR analysis with the appropriate oligonucleotides given in Table 2.
The resulting strain was named *E. coli* MG1655 *Δedd-eda*::Km.

### 1.2. Construction of strain E. coli MG1655 Δedd eda::Km, ΔgloA::Cm

### 1.2.1. Construction of strain E. coli MG1655 ΔgloA::Cm

The *gloA* gene was inactivated in strain *E. coli* MG1655 by inserting a chloramphenicol antibiotic cassette and deleting most of the gene concerned using the technique described in Protocol 1 with the oligonucleotides given in Table 1. The deletion was checked by a PCR analysis with the appropriate oligonucleotides given in Table 2.
The resulting strain was named *E. coli* MG1655 *ΔgloA*::Cm.

### 1.2.2. Construction of a modified strain E. coli MG1655 Δedd eda::Km, ΔgloA:: cam

The deletion of *gloA* gene by replacement of the gene by a chloramphenicol resistance cassette in the strain *E. coli* MG1655 *Δedd-eda::Km* was performed by the technique of transduction with phage P1 (Protocol 2).

### Protocol 2 : Transduction with phage P1 for deletion of a gene

The deletion of the chosen gene by replacement of the gene by a resistance cassette (kanamycin or chloramphenicol) in the recipient *E. coli* strain was performed by the technique of transduction with phage P1. The protocol was in two steps, (i) preparation of the phage lysate on strain MG1655 with a single gene deleted and (ii) transduction of the recipient strain by this phage lysate.

### Preparation of the phage lysate

- Seeding with 100 µl of an overnight culture of the strain MG1655 with a single gene deleted of 10 ml of LB + Cm 30 µg/ml + glucose 0.2% + CaCl₂ 5 mM.
- Incubation for 30 min at 37°C with shaking.
- Addition of 100 µl of phage lysate P1 prepared on the wild type strain MG1655 (approx. 1 x 10⁹ phage/ml).
- Shaking at 37°C for 3 hours until all cells were lysed.
- Addition of 200 µl of chloroform, and vortexing.
- Centrifugation for 10 min at 4500 g to eliminate cell debris.
- Transfer of supernatant in a sterile tube and addition of 200 µl of chloroform.
- Storage of the lysate at 4°C

### Transduction

- Centrifugation for 10 min at 1500 g of 5 ml of an overnight culture of the *E. coli* recipient strain in LB medium.
- Suspension of the cell pellet in 2.5 ml of MgSO₄ 10 mM, CaCl₂ 5 mM.
- Control tubes: 100 µl cells
   100 µl phages P1 of the strain MG1655 with a single gene deleted.
- Tube test: 100 µl of cells + 100 µl phages P1 of strain MG1655 with a single gene deleted.
- Incubation for 30 min at 30°C without shaking.
- Addition of 100 µl sodium citrate 1 M in each tube, and vortexing.
- Addition of 1 ml of LB.
- Incubation for 1 hour at 37°C with shaking
- Plating on dishes LB + Cm 30 µg/ml after centrifugation of tubes for 3 min at 7000 rpm.
- Incubation at 37°C overnight.

The antibiotic-resistant transformants were then selected.

The deletion was checked by a PCR analysis with the appropriate oligonucleotides given in Table 2 as well as the other deletions already present in the strain.

The resulting strain was named *E. coli* MG1655 Δ*edd-eda: :Km, ΔgloA::Cm*

### 1.3. Construction of strain E. coli MG1655 Δedd-eda ΔgloA

The antibiotic resistance cassettes were eliminated in the strain *E. coli* MG1655 *Δedd-eda::Km, ΔgloA::Cm* according to Protocol 3.

### Protocol 3: Elimination of resistance cassettes (FRT system)

The chloramphenicol and/or kanamycin resistance cassettes were eliminated according to the following technique. The plasmid pCP20 carrying the FLP recombinase acting at the FRT sites of the chloramphenicol and/or kanamycin resistance cassettes was introduced into the strain by electroporation. After serial cultures at 42°C, the loss of the antibiotic resistance cassettes was checked by PCR analysis with the oligonucleotides given in Table 2.

The presence of the modifications previously built in the strain was checked using the oligonucleotides given in Table 2.

The strain obtained was named *E. coli MG1655 Δedd-eda ΔgloA.*

### 1.4. Construction of strain E. coli MG1655 Δedd-eda ΔgloA, ΔaldA::Cm

### 1.4.1. Construction of strain E. coli MG1655 ΔaldA::Cm

The *aldA* gene was inactivated in strain *E. coli* MG1655 by inserting a chloramphenicol antibiotic cassette and deleting most of the gene concerned using the technique described in Protocol 1 with the oligonucleotides given in Table 1. The deletion was checked by a PCR analysis with the appropriate oligonucleotides given in Table 2.
The resulting strain was named *E. coli* MG1655 *ΔaldA*::Cm.

### 1.4.2. Construction of strain E. coli MG1655 Δedd-eda ΔgloA, ΔaldA::Cm

The deletion of *aldA* gene by replacement of the gene by a chloramphenicol resistance cassette in the strain *E. coli MG1655 Δedd-eda ΔgloA* was performed by the technique of transduction with phage P1 (Protocol 2).

The *ΔaldA*::Cm deletion and the other modifications were checked using the oligonucleotides described in Table 2.

### 1.5. Construction of strain E. coli MG1655 Δedd-eda ΔgloA, ΔaldA::Cm, ΔaldB::Km

### 1.5.1. Construction of strain E. coli MG1655 ΔaldB::Km

The *aldB* gene was inactivated in strain *E. coli* MG1655 by inserting a Kanamycin antibiotic cassette and deleting most of the gene concerned using the technique described in Protocol 1 with the oligonucleotides given in Table 1. The deletion was checked by a PCR analysis with the appropriate oligonucleotides given in Table 2.
The resulting strain was named *E. coli* MG1655 *ΔaldB*::Km.

### 1.5.2. Construction of strain E. coli MG1655 Δedd-eda ΔgloA, ΔaldA::Cm, ΔaldB::Km

Deletion of the *aldB* gene by replacement of the gene by a Kanamycin resistance cassette in the strain *E. coli MG1655 Δedd-eda ΔgloA ΔaldA*::Cm, was performed by the technique of transduction with phage P1 (Protocol 2).
The *ΔaldB*::Cm deletion and the other modifications were checked using the oligonucleotides described in Table 2.

### 1.6. Construction of strain E. coli MG1655 Δedd-eda ΔgloA, ΔaldA, ΔaldB

The antibiotic resistance cassette was eliminated in strain *E. coli* MG1655 *Δedd-eda ΔgloA, ΔaldA::Cm, ΔaldB*::Km according to Protocol 3.

The loss of the antibiotic resistance cassette was checked by PCR analysis with the oligonucleotides given in Table 2. The presence of the modifications previously built in the strain was also checked using the oligonucleotides given in Table 2.
The strain obtained was named *E. coli* MG1655 *Δedd-eda ΔgloA, ΔaldA, ΔaldB.*

### 1.7. Construction of strain E. coli MG1655 Δedd-eda ΔgloA, ΔaldA, ΔaldB, ΔarcA::Km

### 1.7.1. Construction of strain E. coli MG1655 ΔarcA::Km

The *arcA* gene was inactivated in strain *E. coli* MG1655 by inserting a Kanamycin antibiotic cassette and deleting most of the gene concerned using the technique described in Protocol 1 with the oligonucleotides given in Table 1. The deletion was checked by a PCR analysis with the appropriate oligonucleotides given in Table 2.
The resulting strain was named *E. coli* MG1655 *ΔarcA*::Km.

### 1.7.2. Construction of strain E. coli MG1655 Δedd-eda ΔgloA, ΔaldA, ΔaldB, ΔarcA::Km

Deletion of the *arcA* gene by replacement of the gene by a Kanamycin resistance cassette in the strain *E. coli Δedd-eda ΔgloA, ΔaldA, ΔaldB, ΔarcA*::Km, was performed by the technique of transduction with phage P1 (Protocol 2).
The *ΔarcA*::Km deletion and the other modifications were checked using the oligonucleotides described in Table 2.

### 1.8. Construction of strain E. coli MG1655 Δedd-eda ΔgloA, ΔaldA, ΔaldB, ΔarcA::Km, Δndh::Cm

### 1.8.1. Construction of strain E. coli MG1655 Δndh::Cm

The *ndh* gene was inactivated in strain *E. coli* MG1655 by inserting a chloramphenicol resistance cassette and deleting most of the gene concerned using the technique described in Protocol 1 with the oligonucleotides given in Table 1. The deletion was checked by a PCR analysis with the appropriate oligonucleotides given in Table 2. The resulting strain was named *E. coli* MG1655 *Δndh*::Cm.

### 1.8.2. Construction of strain E. coli MG1655 Δedd-eda ΔgloA, ΔaldA, ΔaldB, ΔarcA::Km, Δndh::Cm

Deletion of the *ndh* gene by replacement of the gene by a chloramphenicol resistance cassette in the strain *E. coli Δedd-eda ΔgloA, ΔaldA, ΔaldB, ΔarcA*::Km, was performed by the technique of transduction with phage P1 (Protocol 2).

The *Δndh*::Cm deletion and the other modifications were checked using the oligonucleotides described in Table 2.

### 1.9. Construction of strain E. coli MG1655 Δedd-eda, ΔgloA, ΔaldA, ΔaldB, ΔarcA, Δndh

The antibiotic resistance cassettes were eliminated in strain *E. coli* MG1655 *Δedd-eda ΔgloA, ΔaldA, ΔaldB, ΔarcA*::Km, *Δndh*::Cm according to Protocol 3.

The loss of the antibiotic resistance cassettes was checked by PCR analysis with the oligonucleotides given in Table 2. The presence of the modifications previously built in the strain was also checked using the oligonucleotides given in Table 2.

The strain obtained was named *E. coli* MG1655 *Δedd-eda ΔgloA, ΔaldA, ΔaldB, ΔarcA, Δndh.*

### 1.10. Construction of strain E. coli MG1655 Δdd-eda, ΔgloA,ΔaldA, ΔaldB, ΔarcA, Δndh pME101VB06-yqhD*(G149E)-gld*(A160T)

### 1.10.1. Construction of plasmid pME101VB06-yqhD*(G149E)-gldA*(A160T)

### 1.10.1.1. Construction of plasmid pME101VB06-gldA*(A160T)

The gldA*(A160T) gene was amplified from plasmid pME101VB01-gldA*(A160T) (construction described in patent application WO2001/012702A1) using the following oligonucleotides :
gldA* sans lacI et op 
ccaatgcatGAGCTGTTGACAATTAATCATCCGGCTCGTATAATGTGTGGtctagatcacac
aggaaacagaccatgggctag (SEQ ID N43)
with :
   - region homologous to pME101VB01-gldA*(A160T) (small letters)
   - *Xba*I restriction site (underlined letters)
   - promoter Ptrc01 (capital letters)
   - *Nsi*I restriction site (bold letter)

PME102seqR
GCGGTATCATCAACAGGCTTACCCG (SEQ ID NO 44)

With: a region homologous to pME101VB01-gldA*(A160T).

The PCR product cut with the restriction enzymes *Nsi*I and *Sac*I was cloned in pME101VB01-gldA*(A160T) treated with the same restriction enzyme. The resulting plasmid was named pME101VB06-gldA*(A160T)

### 1.10.1.2. Construction of plasmid pME101VB06-yqhD*(G149E)-gldA*(A160T)

The pSCB-yqhD*(G149E) plasmid (construction described in patent application W02011/012697A2) was cut with the restriction enzymes *Xba*I and *SnaB*I and the fragment containing *yqhD**(G149E) was cloned into the *Xba*I / *SnaB*I sites of the pME101VB06-gldA*(A160T) plasmid. The resulting plasmid was named pME101VB06-*yqh*D*(G149E)-gldA*(A160T).

### 1.10.2. Construction of strain E. coli MG1655 Δedd-eda, ΔgloA, ΔaldA, ΔaldB, ΔarcA, Δndh pME101VB06-yqhD*(G149E)-gld*(A160T)

The pME101VB06-*yqhD*(G149E)-gldA*(A160T)* plasmid was introduced by electroporation into strain *E. coli* MG1655 Δ*gloA,* Δ*edd-eda,* Δ*aldA,* Δ*aldB,* Δ*arcA,* Δ*ndh.*

The strain obtained was named *E. coli* MG1655 Δ*gloA,* Δ*edd-eda,* Δ*aldA,* Δ*aldB,* Δ*arcA,* Δ*ndh* (pME101VB06-*yqhD*(G149E)-gldA*(A160T).*

### 1.11. Construction of strain E. coli MG1655 mgsA*(H21Q) Δedd-eda, ΔgloA, ΔaldA, ΔaldB, ΔarcA, Δndh (pME101VB06-yqhD*(G149E)-gldA*(A160T).

### 1.11.1. Construction of strain E. coli MG1655 mgsA*(H21Q)::Km

A mutation was introduced in the *mgsA* gene in order to obtain the mutant protein MgsA*(H21Q). The technique used to build this modification was described by Heermann et al. (2008), Microbial Cell Factories.7(14): 1-8.

The following oligonucleotides were used to amplify the *rpsL*-Neo cassette :
1.mgsA*(H21Q) ::rpsL-Neo F, consisting in 105 pb, (SEQ ID NO 45) 
gttaactacggatgtacattatggaactgacgactcgcactttacctgcgcggaaacatattgcgctggtggcacacgatcaggcct
ggtgatgatggcgggatc
 with,
   - a region (underlined letters) homologous to the *E.coli* chromosome from 1026258 to 1026177
   - a region (bold face letters) to amplified rpsL-Neo cassette.
2. mgsA*(H21Q) ::rpsL-Neo R (SEQ ID NO 46) 
gggaaattaagttaccggtagtgcctgtTgcatacagtacgtgttgttccagtaacggttgatgccgttccacccagctcatcagcat
Ctgtttgcattcagaagaactcgtcaagaagg
 with,
   - a region (underlined letters) homologous to the *E. coli* chromosome from 1026079 to 1026175) with two mutation, the first one (bold capital letters) to create the mutation H21Q and the second one (Italic capital letter) to create the restriction site *AlwN*1.
   - a region (bold small letters) to amplified rpsL-Neo cassette.

The fragment obtained was introduced into strain MG1655 *rpsL** (built as described in Heermann et al.) according to Protocol 1. The strain obtained was checked by PCR and sequence analysis. The strain obtained was named *E. coli mgsA**(H21Q)::*rpsL*-Neo. Deletion of the *rpsL*-Neo cassette was performed according to Protocol 1. The fragment transformed was obtained by restriction with *Nco*I and *Sac*I of the pETTOPO-*mgsA**(H21Q) plasmid (described in patent application WO2011012693A1).

The modification was checked by PCR using oligonucleotides described in Table 1. The strain obtained was named strain *E. coli MG1655 mgsA**(H21Q).

### 1.11.2. Construction of strain E. coli MG1655 mgsA*(H21Q)::Km

A kanamycin resistance cassette was introduced in 3' of mgsA*(H21Q) open reading frame using the following oligonucleotides : mgsA ::Km F and mgsA ::Km R

The PCR product was introduced into strain MG1655 *mgsA*(H21Q)* according to Protocol 1. The resulting strain was checked by PCR and named *E. coli mgsA**(H21Q)::*Km*

### 1.11.3. Construction of strain E. coli MG1655 mgsA*(H21Q)::Km ΔgloA, Δedd-eda, ΔaldA, ΔaldB, ΔarcA, Δndh (pME101VB06-yqhD*(G149E)-gldA*(A160T))

Replacement of mgsA with mgsA*(H21Q)::Km into strain *E. coli ΔgloA, Δeed-eda ΔaldA, ΔaldB, ΔarcA, Δndh (pME101VB01-yqhD*(G149E)-gldA*(A160T))* was performed by the technique of transduction with phage P1.

The mgsA*(H21Q)::Km modification and the other modifications were checked using the oligonucleotides described in Table 1.

### 1.11.4. Construction of strain E. coli MG1655 mgsA*(H21Q) ΔgloA, Δedd-eda, ΔaldA, ΔaldB, ΔarcA, Δndh (pME101VB06-yqhD*(G149E)-gldA*(A160T))

The antibiotic resistance cassette was eliminated in strain MG1655 mgsA*(H21Q)::Km *ΔgloA, Δedd-eda, ΔaldA, ΔaldB, ΔarcA, Δndh (pME101VB06-yqhD*(G149E)-gldA *(A160T))* according to Protocol 3.

The presence of the modifications previously built in the strain was checked using the oligonucleotides given in Table 2.

The strain obtained was named *E. coli* MG1655 mgsA*(H21Q) *ΔgloA, Δedd-eda, ΔaldA, ΔaldB, ΔarcA, Δndh (pME101VB06-yqhD*(G149E)-gldA *(A160T)).*

### 1.12. Construction of strain E. coli MG1655 mgsA*(H21Q) CI857-PR01/RBS01*2-gapA::Km Δedd-eda, ΔgloA, ΔaldA, ΔaldB, ΔarcA, Δndh (pME101VB06-yqhD*(G149E)-gldA*(A160T).

Replacement of the *gapA* promoter by the thermoregulated promoter CI857-PR01/RBS01*2-gapA::Km in strain *E. coli* MG1655 mgsA*(H21Q) Δ*gloA*, Δ*edd-eda,* Δ*aldA*, Δ*aldB*, Δ*arcA*, Δ*ndh (pME101VB06-yqhD*(G149E)-gldA*(A160T))* was performed by the technique of transduction with phage P1 (Protocol 2).

The thermoregulated promoter in front of gapA and the other modifications were checked using the oligonucleotides described in Table 2.

The strain obtained was named MG1655 mgsA*(H21Q) CI857-PR01/RBS01*2-gapA::Km Δ*edd-eda*, Δ*gloA*, Δ*aldA*, Δ*aldB*, Δ*arcA*, Δ*ndh* (pME101VB06-*yqhD*(G149E)-gldA*(A160T) .*

### 2. Construction of strain E. coli MG1655 CI857-PR01/RBS01*2-gapA::Km mgsA*(H21Q), Δedd-eda, ΔgloA, ΔaldA, ΔaldB, ΔarcA, Δndh, pME101VB06-yqhD*(G149E)-gldA*(A160T) pBBR1MCS5-cscBKAR

### 2.1. Construction of plasmid pBBR1MCS5-cscBKAR

The pKJL101.1 plasmid (Jahreis et al. (2002), J. Bacteriol. 184:5307-5316) was digested by *EcoR*I. The fragment containing the *cscBKAR* gene was cloned in pBBR1MCS5 (Kovach et al. (1995), Gene, 166 175-176) also digested by *EcoRI.*

The plasmid obtained was named pBBR1MCS5-*cscBKAR*.

### 2.2. Construction of strain E. coli MG1655 CI857-PR01/RBS01*2-gapA::Km mgsA*(H21Q), Δedd-eda, ΔgloA, ΔaldA, ΔaldB, ΔarcA, Δndh, pME101VB06-yqhD*(G149E)-gldA*(A160T) pBBRIMCSS-cscBKAR

The pBBR1MCS5-*cscBKAR* plasmid was introduced by electroporation into strain *E. coli MG1655 CI857-PR01*/*RBS01*2-gapA::Km mgsA*(H21Q),* Δ*edd-eda*, Δ*gloA*, Δ*aldA,* Δ*aldB,* Δ*arcA,* Δ*ndh, pME101VB06-yqhD*(G149E)-gldA*(A160T).*

The strain obtained was named *E. coli MG1655 CI857-PR01*/*RBS01*2-gapA::Km mgsA*(H21Q)*, Δ*edd-eda*, Δ*gloA*, Δ*aldA*, Δ*aldB*, Δ*arcA*, Δ*ndh*, *pME101VB06-yqhD*(G149E)-gldA*(A160T) pBBR1MCS5-cscBKAR.*

### 3. Assessment of 1,2-propanediol production from glucose in isogenic strains differing only in the thermoregulated gapA

Flask cultures were carried out in order to appraise the effect of the construction described above on the growth rate and the production of 1,2-propanediol. A minimal medium (MML11PG1_100, see composition in Table 1) with 10 g/L glucose as sole carbon source was used. The cultures were incubated either at 37°C or 30°C under aerobic conditions. 20 g/L MOPS were added in culture medium in order to maintain a pH above 6.0 throughout fermentation course.

1,2-propanediol (PG), acetol (HA) and glucose were separated and quantified by HPLC. The yield of PG+HA over glucose (Y_{PG+HA}) give an estimate of strain performance for the production of 1,2-propanediol. Indeed, in such culture conditions, conversion of acetol into 1,2-propanediol is not maximal (a microaerobic or anaerobic step would be necessary at the end of the culture to optimize said conversion).

**Table 5: Production yield (Y_{PG+HA}) as determined during aerobic flask cultures on minimal medium**

| **Genotype** | **Temperature** | **Y_{PG+HA} (g/g)** |
|---|---|---|
| MG1655 mgsA*(H21Q) DgloA Dedd+eda DaldA DaldB DarcA Dndh (pME101VB06-yqhD*(G149E)-gldA*(A160T)) Sp | **37°C** | 0.16 |
| | **30°C** | 0.20 |
| MG1655 mgsA*(H21Q) CI857-PR01/RBS01*2-gapA::Km DgloA Dedd+eda DaldA DaldB DarcA Dndh (pME101VB06-yqhD*(G149E)-gldA*(A160T)) Sp | **37°C** | 0.15 |
| | **30°C** | 0.21 |

The results presented in Table 5 show that a higher PG+HA yield over glucose is obtained at 30°C in the strain bearing the thermoregulated promoter in front of *gapA.*

### 4. Assessment of 1,2-propanediol production from sucrose in isogenic strains differing only in the thermoregulated gapA

Flask cultures were carried out in order to appraise the effect of the construction described above on the growth rate and the production of 1,2-propanediol. A minimal medium (MML11PG1_100, see composition in Table 3) with 10 g/L sucrose as sole carbon source was used. The cultures were incubated either at 37°C or 30°C under aerobic conditions. 20 g/L MOPS were added in culture medium in order to maintain a pH above 6.0 throughout fermentation course.

1,2-propanediol (PG), acetol (HA) and sucrose were separated and quantified by HPLC. The growth rate (µ) and the yield of PG+HA over sucrose (Y_{PG+HA}) give an estimate of strain performance for the production of 1,2-propanediol. Indeed, in such culture conditions, conversion of acetol into 1,2-propanediol is not maximal (a microaerobic or anaerobic step would be necessary at the end of the culture to optimize said conversion).

**Table 6: Growth rate (µ) and production yield (Y_{PG+HA}) as determined during aerobic flask cultures on minimal medium**

| **Genotype** | **Temperature** | **µ (h⁻¹)** | **Y_{PG+HA} (g/g)** |
|---|---|---|---|
| MG1655 mgsA*(H21Q) DgloA Dedd+eda DaldA DaldB DarcA Dndh (pME101VB06-yqhD*(G149E)-gldA*(A160T)) Sp (pBBR1MCS5-cscBKAR) Gt | **37°C** | 0.23 | 0.22 |
| | **30°C** | 0.16 | 0.26 |
| MG1655 mgsA*(H21Q) CI857-PR01/RBS01*2-gapA::Km DgloA Dedd+eda DaldA DaldB DarcA Dndh (pME101VB06-yqhD*(G149E)-gldA*(A160T)) Sp (pBBR1MCS5-cscBKAR) Gt | **37°C** | 0.33 | 0.26 |
| | **30°C** | 0.20 | 0.34 |

The results presented in Table 6 show that whatever the temperature is, higher growth rates and PG+HA yield over sucrose are obtained in the strain bearing the thermoregulated promoter in front of *gapA*.

### Example 4: Comparison of CI857-PR01/RBS01*2 gapA and C1857-PR01/RBS11 gapA in a 1,2-propanediol production strain using sucrose as a carbon source : construction of an E. coli 1,2-propanediol producing strain with thermoregulated promoter C1857-PR01/RBS11-gapA.

### 1. Construction of strain E. coli MG1655 C1857-PR01/RB11-gapA::Km mgsA*(H21Q), Δedd-eda, ΔgloA, ΔaldA, ΔaldB, ΔarcA, Δndh, pME101VB06-yqhD*(G149E)-gldA*(A160T) pBBR1MCS5-cscBKAR

### 1.1. Construction of strain E. coli MG1655 CI857-PR01/RBS11-gapA::Km mgsA*(H21Q) Δedd-eda, ΔgloA, ΔaldA, ΔaldB, ΔarcA, Δndh (pME101VB06-yqhD*(G149E)-gldA*(A160T).

### 1.1.1. Construction of strain E. coli MG1655 mgsA*(H21Q) Δedd-eda, ΔgloA, ΔaldA, ΔaldB, ΔarcA, Δndh (pME101VB06-yqhD*(G149E)-gldA*(A160T)

Construction of strain *E. coli* MG1655 *mgsA**(H21Q) Δ*edd*-*eda*, Δ*gloA*, Δ*aldA*, Δ*aldB*, Δ*arcA*, Δ*ndh* (pME101VB06-*yqhD*(G149E)-gldA*(A160T)* was described in Example 3.

### 1.2.2. Construction of strain E. coli MG1655 CI857-PR01/RBS11-gapA::Km mgsA*(H21Q), Δedd-eda, ΔgloA, ΔaldA, ΔaldB, ΔarcA, Δndh, pME101VB06-yqhD*(G149E)-gldA*(A160T)

Replacement of the *gapA* wild-type promoter by the thermoregulated promoter CI857-PR01/RBS11-*gapA*::Km in strain *E. coli* MG1655 mgsA*(H21Q) Δ*gloA*, Δ*edd-eda*, Δ*aldA*, Δ*aldB*, Δ*arcA*, Δ*ndh* (pME101VB06-*yqhD**(G149E)-*gldA**(A160T)) was performed by the technique of transduction with phage P1 (Protocol 2).

The thermoregulated promoter in front of *gapA* and the other modifications were checked using the oligonucleotides described in Table 2.

### 1.2. Construction of strain E. coli MG1655 CI857-PR01/RB11-gapA::Km mgsA*(H21Q), Δedd-eda, ΔgloA, ΔaldA, ΔaldB, ΔarcA, Δndh, pME101VB06-yqhD*(G149E)-gldA*(A160T) pBBR1MCS5-cscBKAR

### 1.2.1. Construction of plasmid pBBR1MCS5-cscBKAR

Construction of the pBBR1MCS1-*cscBKAR* plasmid was described in Example 3.

### 1.2.2. Construction of strain E. coli MG1655 CI857-PR01/RBS01*2-gapA::Km mgsA*(H21Q), Δedd-eda, ΔgloA, ΔaldA, ΔaldB, ΔarcA, Δndh, pME101VB06-yqhD*(G149E)-gldA*(A160T) pBBR1MCS5-cscBKAR

The pBBRIMCS5-*cscBKAR* plasmid was introduced by electroporation into strain *E. coli MG1655 CI857-PR01*/*RBS11-gapA::Km mgsA*(H21Q),* Δ*edd-eda,* Δ*gloA,* Δ*aldA,* Δ*aldB,* Δ*arcA,* Δ*ndh, pME101VB06-yqhD*(G149E)-gldA*(A160T).*

The strain obtained was named *E. coli MG1655 CI857-PR01*/*RBS11-gapA::Km mgsA*(H21Q),* Δ*edd-eda,* Δ*gloA,* Δ*aldA,* Δ*aldB,* Δ*arcA,* Δ*ndh, pME101VB06-yqhD*(G149E)-gldA*(A160T) pBBR1MCS5-cscBKAR.*

### 2. Construction of strain E. coli MG1655 CI857-PR01/RBS01*2-gapA mgsA*(H21Q), Δedd-eda, ΔgloA, ΔaldA, ΔaldB, ΔarcA, Δndh, pME101VB06-yqhD*(G149E)-gldA*(A160T) pBBR1MCS5-cscBKAR

The antibiotic resistance cassette was eliminated in strain *E. coli MG1655* CI857-PR01/RBS01*2-*gapA::Km mgsA*(H21Q),* Δ*edd-eda,* Δ*gloA,* Δ*aldA,* Δ*aldB,* Δ*arcA,* Δ*ndh, pME101VB06-yqhD*(G149E)-gldA*(A160T)* pBBRIMCS5-*cscBKAR* according to Protocol 3.

The loss of the antibiotic resistance cassette was checked by PCR analysis with the oligonucleotides given in Table 2. The presence of the modifications previously built in the strain was also checked using the oligonucleotides given in Table 2.The presence of plasmid was validated by extraction and restriction.

The strain obtained was named *E. coli* MG1655 Δ*edd-eda* Δ*gloA*, Δ*aldA*, Δ*aldB. E. coli MG1655* CI857-PR01/RBS01*2-*gapA mgsA*(H21Q),* Δ*edd-eda*, Δ*gloA*, Δ*aldA*, Δ*aldB*, Δ*arcA*, Δ*ndh*, *pME101VB06-yqhD*(G149E)-gldA*(A160T)* pBBR1MCS5-*cscBKAR.*

### 3. Assessment of 1,2-propanediol production from sucrose in isogenic strains differing only by the thermoregulated gapA

Two cultures at 37°C were carried out in 500 ml instrumented bioreactor in order to appraise the effect of the construction described above on the growth rate and the production of 1,2-propanediol. Inocula were obtained after 24 h precultures at 37°C in Erlenmeyer Flasks (minimal medium MML11PG1_100, see composition in Table 3, with 20 g/l sucrose, 0.25 g/l LB and 40 g/l MOPS,). Bioreactors with 200 ml of minimal medium (MML11PG1_122, idem MML11PG1_100 with a doubled concentration of PO4) with 20 g/l sucrose as sole carbon source were inoculated with 0.2 g/l biomass. pH was maintained at 6.8 using a 10 % NH4OH solution and pO2 was regulated at 30 % by modulating the agitation with a fixed airflow of 40 Nl/h. A fed-batch feeding (FedPG01_V13, see composition in Table 7) with pulse additions of 10 g/l sucrose was applied in order to maintain an excess of sucrose throughout fermentation course.

**Table 7: Minimal medium composition (all concentration expressed in g/l)**

| | |
|---|---|
| Saccharose | 500 |
| (NH4)2SO4 | 15 |
| MgSO4 | 1 |
| Fe(III) citrate, H2O | 0,0424 |
| MnCl2, 4H2O | 0,0120 |
| Zn(CH3COO)2, 2H2O | 0,0104 |
| Fe(III) citrate, H2O | 0,0100 |
| Thiamine, HCl | 0,01 |
| EDTA, 2Na, 2H2O (C10H16N2O8, 2Na, 2H2O) | 0,0067 |
| H3BO3 | 0,0024 |
| CoCl2, 6H2O | 0,0020 |
| Na2MoO4, 2H2O | 0,0020 |
| CuCl2, 2H2O | 0,0012 |

1,2-propanediol (PG), acetol (HA) and sucrose were separated and quantified by HPLC. The yield of PG+HA over sucrose (YPG+HA) gives an estimate of strain performance for the production of 1,2-propanediol. Indeed in such culture conditions conversion of acetol to 1,2-propanediol is not maximal (a microaerobic or anaerobic step would be necessary at the end of the culture).

**Table 8: Growth rate (µ) and production yield (YPG+HA) as determined during aerobic flask cultures on minimal medium**

| **Genotype** | **Y_{PG+HA} (g/g)** |
|---|---|
| MG1655 mgsA*(H21Q) CI857-PR01/RBS01*2-gapA DgloA Dedd+eda DaldA DaldB DarcA Dndh (pME101VB06-yqhD*(G149E)-gldA*(A160T)) Sp (pBBR1MCS5-cscBKAR) Gt | 0.22 |
| MG1655 mgsA*(H21Q) CI857-PR01/RBS11-gapA::Km DgloA Dedd+eda DaldA DaldB DarcA Dndh (pME101VB06-yqhD*(G149E)-gldA*(A160T)) Sp (pBBR1MCS5-cscBKAR) Gt | 0.25 |

The results presented in Table 8, and Table 6, show that the use of artificial RBS (RBS01*2 or RBS11) leads to an improved yield of PG+HA over sucrose. Moreover these results show that a higher PG+HA yield over sucrose is obtained in the strain bearing the CI857-PR01/RBS11 promoter in front of *gapA*, compared to the strain with the CI857-PR01/RBS01*2 promoter.

### Example 5: Improvement of Methylglyoxal Reductase activity

### 1. Construction of strain E. coli MG1655 CI857-PR01/RBS01*2-gapA::Km mgsA*(H21Q), Δedd-eda, ΔgloA, ΔaldA, ΔaldB, ΔarcA, Δndh, pME101VB06-yafB-gldA*(A160T) pBBR1MCS5-cscBKAR

### 1.1. Construction of plasmid pME101VB17-yafB-gldA*(A160T)

The *yafB* gene was PCR amplified from genomic DNA of *E. coli* MG1655 using the following oligonucleotides :
RBS23-yafB F (SEQ ID NO 47) 
TCTAGAGTAGTCACTACTACGCGACCTAGGAGGTTCTTTatggctatccctgcatttggtttag
g
With
   - XbaI restriction site (bold capital letters)
   - ribosome binding site region (capital letters)
   - Homologous region to *E. coli* chromosome from 229167 to 229192 (underlined letters)
yafB R (SEQ ID NO 48)
TACGTA**gctagc**ttaatcccattcaggagccagac

With
- *Nhe*I restriction site (small letters)
- *SnaB*I restriction site (bold capital letters)
- Homologous region to *E. coli* chromosome from 229970 to 229948 (underlined letters)

The PCR product obtained was digested by *Xba*I and *SnaB*I and cloned in the pME101VB18-gldA*(A160T) digested with the same restriction enzyme. The plasmid obtained was named pME101VB17-*yafB-gldA**(A160T).

### 1.2. Construction of strain E. coli MG1655 CI857-PR01/RBS01*2-gapA::Km mgsA*(H21Q), Δedd-eda, ΔgloA, ΔaldA, ΔaldB, ΔarcA, Δndh, pME101VB17-yafB-gldA*(A160T) pBBR1MCS5-cscBKAR

The presence of *mgsA**(H21Q) and DgloA was toxic in our modified strain. To counteract this phenomenon, the over expression of Methylglyoxal Reductase enzymes must be permanently maintained in this strain. To change a plasmid by the same one but with other genes, an intermediary plasmid with a different antibiotic resistance needs to be used.

### 1.2.1. Construction of plasmid pME101VB09-yqhD*(G149E)-gldA*(A160T)

The Ampicillin resistance cassette was PCR amplified from pLox/Ap (Genebridges®) using the following oligonucleotides :
Ap/lox F (SEQ ID NO 49)
tccCCCGGG**aattaaccctcactaaagggcggccgc**
with
   - homology region to pLox/Ap (small bold letters)
   - SmaI restriction site (Capital letters)
Ap/lox R (SEQ ID NO 50)
tccCCCGGG**taatacgactcactatagggctcgag**
with
   - homology region to pLox/Ap (small bold letters)
   - SmaI restriction site (Capital letters)

The PCR product was digested by *Sma*I and cloned in pME101VB06-*yqhD**(G149E)-*gldA**(A160T) digested by *Nae*I. The plasmid obtained was named pME101VB09-*yqhD**(G149E)-*gldA**(A160T).

### 1.2.2. Construction of strain E. coli MG1655 CI857-PR01/RBS01*2-gapA:: Km mgsA*(H21Q), Δedd-eda, ΔgloA, ΔaldA, ΔaldB, ΔarcA, Δndh, pME101VB09-yqhD*(G149E)-gldA*(A160T) pBBR1MCS5-cscBKAR

Swapping of pME101VB06-yqhD**(G149E)*-gldA**(A160T)* by pME101VB09-*yqhD**(G149E)-*gldA**(A160T) in strain *E. coli* MG1655 CI857-PR01/RBS01*2-*gapA*::Km *mgsA*(H21Q),* Δ*edd-eda,* Δ*gloA,* Δ*aldA,* Δ*aldB,* Δ*arcA,* Δ*ndh*, pME101VB06-*yqhD**(G149E)-*gldA**(A160T) *pBBR1MCS5-cscBKAR* (see Example 3) was done according to Protocol 4.

### Protocol 4

The modified strain containing plasmid 1 was electroporated with plasmid 2. Plasmid 1 and plasmid 2 possess the same origin of replication but a different antibiotic resistance cassette. The electroporated strain was selected on the antibiotic corresponding to plasmid 2. One clone was inoculated in LB liquid adding antibiotic corresponding to plasmid 2. After 24h, an isolation and a phenotypic test was done on the obtaincolonies to check for the loss of plasmid 1. If this test was validated, all the modifications of the strain were checked. If not, a new LB culture was inoculated using the first one and a new cycle was done.

The strain obtained was named *E. coli* MG1655 CI857-PR01/RBS01*2-*gapA*::Km *mgsA**(H21Q), Δ*edd-eda*, Δ*gloA*, Δ*aldA*, Δ*aldB*, Δ*arcA*, Δ*ndh*, pME101VB09-*yqhD**(G149E)-*gldA**(A160T) pBBR1MCS5-*cscBKAR.*

### 1.2.3. Construction of strain E. coli MG1655 CI857-PR01/RBS01*2-gapA::Km mgsA*(H21Q), Δedd-eda, ΔgloA, ΔaldA, ΔaldB, ΔarcA, Δndh, pME101VB17-yafB-gldA*(A160T) pBBR1MCS5-cscBKAR.

Swapping of pME101VB09- *yqhD***(G149E)-gldA*(A160T)* by pME101VB18-*yafB-gldA**(A160T) in strain *E. coli MG1655* CI857-PR01/RBS01*2-*gapA*::Km *mgsA**(H21Q), Δ*edd-eda*, Δ*gloA*, Δ*aldA*, Δ*aldB*, Δ*arcA*, Δ*ndh*, pME101VB06-*yqhD**(G149E)-*gldA**(A160T) pBBR1MCS5-*cscBKAR* was done according to Protocol 4.

The strain obtained was named *E. coli* MG1655 CI857-PR01/RBS01*2-*gapA*::Km *mgsA***(H21Q)*, Δ*edd-eda*, Δ*gloA*, Δ*aldA*, Δ*aldB*, Δ*arcA*, Δ*ndh*, pME101VB17-*yafB-gldA*(A160T) pBBR1MCS5-cscBKAR.*

### 2. Construction of strain E. coli MG1655 CI857-PR01/RBS01*2-gapA::Km mgsA*(H21Q), Δedd-eda, ΔgloA, ΔaldA, ΔaldB, ΔarcA, Δndh, pME101VB06-yqhD*(G149E)-yafB-gldA*(A160T) pBBR1MCS5-cscBKAR

### 2.1. Construction of plasmid pME101VB06-yqhD*(G149E)-yafB-gldA*(A160T)

The *yafB* gene was PCR amplified from MG1655 genomic DNA using the following oligonucleotides :
SnaBI-RBS09-yafB F (SEQ ID NO 51) tcc**TACGTA**tcacacaggaaacagaccATGGCTATCCCTGCATTTGG With
   - A SnaBI restriction site (bold capital letters)
   - A ribosome binding site region (Small letters)
   - Homologous region to *E. coli* chromosome from 229167 to 229192 (capital letters)
BglII-yafB R (SEQ ID NO 52) gga**AGATCT**TTAATCCCATTCAGGAGCCAG
With :
   - a BglII restriction site (bold capital letters)
   - Homologous region to *E. coli* chromosome from 229970 to 229948 (capital letters)

The PCR product was digested by *SnaB*I and *Bgl*II and cloned in the pME101VB06-*yqhD**(G149E)-*gldA**(A160T) digested with the same restriction enzymes. The plasmid obtained was named pME101VB06-*yqhD**(G149E)-*yafB*-*gldA**(A160T).

### 2.2. Construction of strain E. coli MG1655 CI857-PR01/RBS01*2-gapA::Km mgsA*(H21Q), Δedd-eda, ΔgloA, ΔaldA, ΔaldB, ΔarcA, Δndh, pME101VB06-yqhD*(G149E)-yafB-gldA*(A160T) pBBR1MCS5-cscBKAR

Swapping of pME101VB09-*yqhD*(G149E)-gldA*(A160T)* by pME101VB06-*yqhD**(G149E)-*yafB-gldA**(A160T) in strain *E*. *coli* MG1655 CI857-PR01/RBS01*2-*gapA*::Km *mgsA**(H21Q), Δ*edd-eda,* Δ*gloA,* Δ*aldA,* Δ*aldB,* Δ*arcA,* Δ*ndh,* pME101VB06-*yqhD**(G149E)-*gldA**(A160T) pBBR1MCS5-*cscBKAR* was done according to Protocol 4.

The strain obtained was named *E*. *coli* MG1655 CI857-PR01/RBS01*2-*gapA*::Km *mgsA**(H21Q), Δ*edd-eda,* Δ*gloA,* Δ*aldA,* Δ*aldB,* Δ*arcA,* Δ*ndh,* pME101VB06-*yqhD**(G149E)-*yafB-gldA**(A160T) pBBR1MCS5-*cscBKAR*.

### Example 6: Improvement of Methylglyoxal Synthase activity : construction of strain E. coli MG1655 CI857-PR01/RBS01*2-gapA::Km mgsA*(H21Q), Δedd-eda, ΔgloA, ΔaldA, ΔaldB, ΔarcA, Δndh, pME101VB06-yqhD*(G149E)-yafB-gldA*(A160T) pBBR1MCS5-cscBKAR pCC1BAC- PmgsA-mgsA*(H21Q)

### 1. Construction of plasmid pCC1BAC- PmgsA-mgsA*(H21Q)

### 1.1. Construction of plasmid pCL1920- PmgsA-mgsA*(H21Q)

The *mgsA**(H21Q) gene with its natural promoter was PCR amplified from genomic DNA of *E*. *coli* MG1655 mgsA*(H21Q)::Km Δ*gloA*, Δ*edd-eda*, Δ*aldA*, Δ*aldB,* Δ*arcA*, Δ*ndh* pME101VB06-*yqhD**(G149E)-*gldA**(A160T) using the following oligonucleotides.

amont mgsA F, homologous to the *E*. *coli* chromosome from 1026734 to 1026715 (SEQ ID NO 53) : CCCAGCTCATCAACCAGGTC

helD F, homologous to the E. coli chromosome from 1025242to 1025260 (SEQ ID NO 54) : CAGCAGAACCCAGGCCAGC
The PCR product was phosphorylated and cloned into the plasmid pCL1920 (Lerner & Inouye, 1990) digested by *Hinc*II and *Sma*I.
The plasmid obtained was named pCL1920-P*mgsA*-*mgsA**(H21Q). The *mgsA**(H21Q) gene was oriented as the Spectinomycin resistance cassette.

### 1.2 Construction of plasmid pCC1BAC- PmgsA-mgsA*(H21Q)

The pCL1920-P*mgsA*-*mgsA**(H21Q) plasmid was digested by EcoRI and HindIII. The fragment containing *PmgsA*-*mgsA**(H21Q) was cloned in pCCIBAC digested with the same restriction enzyme. The plasmid obtained was named pCC1BAC- P*mgsA-mgsA**(H21Q).

### 2. Construction of strain E. coli MG1655 CI857-PR01/RBS01*2-gapA::Km mgsA*(H21Q), Δedd-eda, ΔgloA, ΔaldA, ΔaldB, ΔarcA, Δndh, pME101VB06-yqhD*(G149E)-yafB-gldA*(A160T) pBBR1MCS5-cscBKAR pCC1BAC- PmgsA-mgsA*(H21Q).

The pCC1BAC- P*mgsA-mgsA**(H21Q) plasmid was introduced by electroporation in strain *E. coli* MG1655 CI857-PR01/RBS01*2-*gapA*::Km *mgsA**(H21Q), Δ*edd-eda*, Δ*gloA,* Δ*aldA,* Δ*aldB,* Δ*arcA,* Δ*ndh,* pME101VB06-*yqhD**(G149E)-*yafB-gldA**(A160T) pBBR1MCS5-*cscBKAR*.
The strain obtained was named *E*. *coli* MG1655 CI857-PR01/RBS01*2-*gapA*::Km *mgsA**(H21Q), Δ*edd-eda*, Δ*gloA*, Δ*aldA*, Δ*aldB*, Δ*arcA*, Δ*ndh*, pME101VB06-*yqhD**(G149E)-*yafB*-*gldA**(A160T) pBBR1MCS5-*cscBKAR pCC1BAC- PmgsA-mgsA**(H21Q).

### REFERENCES in the order of citation in the text

1. Badia J, Ros J, Aguilar J (1985), J. Bacteriol. 161: 435-437.
2. Altaras NE and Cameron DC (1999), Appl. Environ. Microbiol. 65: 1180-1185
3. Cameron DC, Altaras NE, Hoffman ML, Shaw AJ (1998), Biotechnol. Prog. 14: 116-125
4. Altaras NE and Cameron DC (2000), Biotechnol. Prog. 16: 940-946
5. Huang K, Rudolph FB, Bennett GN (1999), Appl. Environ. Microbiol. 65: 3244-3247
6. Berrios-Rivera SJ, San KY, Bennett GN (2003), J. Ind. Microbiol. Biotechnol. 30: 34-40
7. Anderson EH (1946), Proc. Natl. Acad. Sci. USA 32: 120-128
8. Miller JH (1992), A Short Course in Bacterial Genetics: A Laboratory Manual and Handbook for Escherichia coli and Related Bacteria, Cold Spring Harbor Laboratory Press, Cold Spring Harbor, New York
9. Schaefer U, Boos W, Takors R, Weuster-Botz D., (1999), Anal. Biochem. 270: 88-96
10. Branlant G, Flesch G, Branlant C., (1983), Gene, 25: 1-7
11. Tang X, Tan Y, Zhu H, Zhao K, Shen W., (2009), Appl Environ Microbiol. Mar; 75:1628-34.
12. Ptashne M., (1967), Proc Natl Acad Sci U S A., 57: 306-313
13. Sussman R, Jacob F. C. R., (1962) Hebd. Seances Acad. Sci. 254: 1517-1519
14. Roberts JW & Roberts CW, (1975), PNAS, 72: 147-151
15. Pabo CO et al., (1979), PNAS, 76: 1608-1612
16. Winstanley C et al., (1989), Applied and Environmental Microbiology, 55: 771-777
17. Ndjonka D & Bell CE, (2006), J. Mol. Biol., 362: 479-489
18. Gimble FS & Sauer RT, (1985), J. Bacteriol., 162: 147-154
19. Gimble FS & Sauer RT, (1986), J. Mol. Biol., 192: 39-47
20. Mermet-Bouvier P & Chauvat F, (1994), Current Microbiology, 28: 145-148
21. Datsenko KA & Wanner BL, (2000), Proc Natl Acad Sci U S A., 97: 6640-6645
22. Lerner CG & Inouye M, (1990), Nucleic Acids Res., 18: 4631 - GenBank AX085428.

## Claims

1. A method for the production of 1,2-propanediol in a fermentative process comprising the following steps:
- culturing a modified microorganism in an appropriate culture medium comprising a source of carbon,
- modulating in said microorganism the level of expression of the gene *gapA* via an external stimulus, and
- recovering 1,2-propanediol from the culture medium,
wherein in said modified microorganism, the expression of the gene *gapA* coding for the enzyme GAPDH is under the control of a heterologous inducible promoter.

2. The method of claim 1 wherein the external stimulus is a physical stimulus.

3. The method of claim 2 wherein the physical stimulus is the temperature of the culture medium.

4. The method according to claim 3 wherein the inducible promoter is selected among:
- promoters regulated by a modified repressor of phage lambda such as:
■ the promoter PR or a derivative of said promoter PR,
■ the promoter PL or a derivative of said promoter PL,
- a modified lac promoter regulated by a temperature sensitive Lac repressor.

5. The method of claim 4 wherein said modified repressor of phage lambda is the lambda repressor allele CI857.

6. The method of claim 5 wherein said modified repressor of phage lambda is a mutant form resistant to cleavage.

7. The method of claim 6 wherein the mutation E117K in the lambda repressor allele CI857 is suppressed.

8. The method of claim 6 or 7 wherein in the mutant form of the repressor allele CI857, the cleavage site between the amino acids A111 and G112 has been suppressed.

9. The method of claim 6 or 7 wherein the mutant form of the repressor allele CI857 comprises at least one mutation chosen among: G147D, G124D, D125N, D125V, D125Y, G185N, G185E, F189L, E127K, and T122I.

10. The method of anyone of claims 5 to 10 wherein in the modified microorganism, the gene *recA* is deleted.

11. The method of anyone of preceding claims wherein the Ribosome Binding Site upstream of the *gapA* gene is mutated.

12. The method of claim 11 wherein the Ribosome Binding Site upstream the *gapA* gene has a nucleotidic sequence chosen among SEQ ID NO 2 and SEQ ID NO 3.

13. The method of anyone of claims 1 to 12 wherein in the modified microorganism, the gene gapA is expressed at a temperature between 37°C to 42°C and has a decreased expression at 30°C.

14. The method of anyone of claims 1 to 13 wherein the modified microorganism is furthermore genetically modified in order to improve the production of 1,2-propanediol.

15. A microorganism modified for an improved production of 1,2-propanediol, wherein in said modified microorganism the expression of the gene *gapA* is under the control of a heterologous inducible promoter as defined in anyone of claims 1 to 12.


SEQUENCE LISTING

<110>  METABOLIC EXPLORER
       Renaud, Stephanie
       Voelker, François
       Figge, Rainer

<120>  Use of inducible promoters in the fermentative production of
       1,2-propanediol

<130>  D29548

<160>  54

<170>  PatentIn version 3.5

<210>  1
<211>  15
<212>  DNA
<213>  artificial sequence

<220>
<223>  Wild type sequence of RBSgapA

<400>  1
tagctggtgg aatat                                                        15


<210>  2
<211>  14
<212>  DNA
<213>  artificial sequence

<220>
<223>  Sequence of RBS01*2

<400>  2
taaggaggtt ataa                                                         14


<210>  3
<211>  14
<212>  DNA
<213>  artificial sequence

<220>
<223>  Sequence of RBS11

<400>  3
taaggagatt ataa                                                         14


<210>  4
<211>  32
<212>  DNA
<213>  artificial sequence

<220>
<223>  Oligonucleotide

<400>  4
tcagccaaac gtctcttcag gccactgact ag                                     32


<210>  5
<211>  115
<212>  DNA
<213>  artificial sequence

<220>
<223>  Oligonucleotide

<400>  5
atgtcagaac gtttctgagc agcacggaaa acaatgcgac cgatacggcc aaaaccgttg      60

atacctactt tgatagtcat ttataacctc cttagtacat gcaaccatta tcacc         115


<210>  6
<211>  114
<212>  DNA
<213>  artificial sequence

<220>
<223>  Oligonucleotide

<400>  6
atgtcagaac gtttctgagc agcacggaaa acaatgcgac cgatacggcc aaaaccgttg      60

atacctactt tgatagtcat ttataatctc cttagtacat gcaaccatta tcac          114


<210>  7
<211>  100
<212>  DNA
<213>  artificial sequence

<220>
<223>  Oligonucleotide

<400>  7
ctcacatctc actttaatcg tgctcacatt acgtgactga ttctaacaaa acattaacac      60

caactggcaa aattttgtcc catatgaata tcctccttag                          100


<210>  8
<211>  99
<212>  DNA
<213>  artificial sequence

<220>
<223>  Oligonucleotide

<400>  8
agtcagtggc ctgaagagac gtttggctga taaaaaaaat aagagttacc atttaaggta      60

actcttattt ttagggccct gtaggctgga gctgcttcg                            99


<210>  9
<211>  25
<212>  DNA
<213>  artificial sequence

<220>
<223>  Oligonucleotide

<400>  9
ctcacatctc actttaatcg tgctc                                           25


<210>  10
<211>  48
<212>  DNA
<213>  artificial sequence

<220>

<223>  Oligonucleotide

<400>  10
ggcagagatg ttctcacctg aactccgtac ctttaccaaa ggtgatgc                    48


<210>  11
<211>  48
<212>  DNA
<213>  artificial sequence

<220>
<223>  Oligonucleotide

<400>  11
gcatcacctt tggtaaaggt acggagttca ggtgagaaca tctctgcc                    48


<210>  12
<211>  23
<212>  DNA
<213>  artificial sequence

<220>
<223>  Oligonucleotide

<400>  12
atgtcagaac gtttctgagc agc                                               23


<210>  13
<211>  100
<212>  DNA
<213>  artificial sequence

<220>
<223>  Oligonucleotide

<400>  13
gcttagcgcc ttctacagct tcacgcgcca gcttagtaat gcggtcgtaa tcgcccgctt      60

ccagcgcatc tgccggaacc catatgaata tcctccttag                            100


<210>  14
<211>  102
<212>  DNA
<213>  artificial sequence

<220>
<223>  Oligonucleotide

<400>  14
cgcgcgagac tcgctctgct tatctcgccc ggatagaaca agcgaaaact tcgaccgttc      60

atcgttcgca gttggcatgc ggtgtaggct ggagctgctt cg                         102


<210>  15
<211>  101
<212>  DNA
<213>  artificial sequence

<220>
<223>  Oligonucleotide

<400>  15
ttaagactgt aaataaacca cctgggtctg cagatattca tgcaagccat gtttaccatc      60

tgcgccgcca ataccggatt tcatatgaat atcctcctta g                    101

<210>  16
<211>  102
<212>  DNA
<213>  artificial sequence

<220>
<223>  Oligonucleotide

<400>  16
atgtcagtac ccgttcaaca tcctatgtat atcgatggac agtttgttac ctggcgtgga      60

gacgcatgga ttgatgtggt agtgtaggct ggagctgctt cg                        102

<210>  17
<211>  101
<212>  DNA
<213>  artificial sequence

<220>
<223>  Oligonucleotide

<400>  17
atgaccaata atcccccttc agcacagatt aagcccggcg agtatggttt ccccctcaag      60

ttaaaagccc gctatgacaa ctgtaggctg gagctgcttc g                         101

<210>  18
<211>  100
<212>  DNA
<213>  artificial sequence

<220>
<223>  Oligonucleotide

<400>  18
tcagaacagc cccaacggtt tatccgagta gctcaccagc aggcacttgg tttgctggta      60

atgctccagc atcatcttgt catatgaata tcctccttag                           100

<210>  19
<211>  98
<212>  DNA
<213>  artificial sequence

<220>
<223>  Oligonucleotide

<400>  19
ccccgcacat tcttatcgtt gaagacgagt tggtaacacg caacacgttg aaaagtattt      60

tcgaagcgga aggctatgtg taggctggag ctgcttcg                              98

<210>  20
<211>  99
<212>  DNA
<213>  artificial sequence

<220>
<223>  Oligonucleotide

<400>  20
ccagatcacc gcagaagcga taaccttcac cgtgaatggt ggcgatgatt tccggcgtat      60

ccggcgtaga ttcgaaatgc atatgaatat cctccttag                              99

<210>  21
<211>  99
<212>  DNA
<213>  artificial sequence

<220>
<223>  Oligonucleotide

<400>  21
ctctcacaaa ttcgctcaaa taataaacaa taaactctgt tttttgatct cacccggtaa      60

agtcgcctat cttttcagct gtaggctgga gctgcttcg                              99

<210>  22
<211>  100
<212>  DNA
<213>  artificial sequence

<220>
<223>  Oligonucleotide

<400>  22
gcaacttcaa acgcggacgg ataacgcggt taatactccc caccagcatc attaatccgg      60

ttttaaagta accatgcagc catatgaata tcctccttag                            100

<210>  23
<211>  100
<212>  DNA
<213>  artificial sequence

<220>
<223>  Oligonucleotide

<400>  23
tccagtcgcc gcatttcaac gacgcggtcg atattctgat ccccgattat cagcgttatc      60

tcgcggaccg tctgaagtaa tgtaggctgg agctgcttcg                            100

<210>  24
<211>  100
<212>  DNA
<213>  artificial sequence

<220>
<223>  Oligonucleotide

<400>  24
tgtggaaata ctgaaaaatc tggatgtgcc ggtggcgaga aaaccgtaag aaacaggtgg      60

cgtttgccac ctgtgcaata catatgaata tcctccttag                            100

<210>  25
<211>  100
<212>  DNA
<213>  artificial sequence

<220>
<223>  Oligonucleotide

<400>  25

atgcgtcttc ttcataccat gctgcgcgtt ggcgatttgc aacgctccat cgatttttat 60

accaaagtgc tgggcatgaa tgtaggctgg agctgcttcg 100

<210> 26
<211> 100
<212> DNA
<213> artificial sequence

<220>
<223> Oligonucleotide

<400> 26
ttagttgccc agaccgcgac cggcgtcttt ctcttcgatt aactcaattt tgtaaccgtc 60

cggatcttcc acaaacgcga catatgaata tcctccttag 100

<210> 27
<211> 24
<212> DNA
<213> artificial sequence

<220>
<223> Oligonucleotide

<400> 27
ccacatgata ccgggatggt gacg 24

<210> 28
<211> 29
<212> DNA
<213> artificial sequence

<220>
<223> Oligonucleotide

<400> 28
gggtagactc cattactgag gcgtgggcg 29

<210> 29
<211> 25
<212> DNA
<213> artificial sequence

<220>
<223> Oligonucleotide

<400> 29
cgttatctct cactactcac tggcg 25

<210> 30
<211> 26
<212> DNA
<213> artificial sequence

<220>
<223> Oligonucleotide

<400> 30
cctccgcctc tttcactcat taagac 26

<210> 31
<211> 40

<212>  DNA
<213>  artificial sequence

<220>
<223>  Oligonucleotide

<400>  31
catatttccc tcaaagaata taaaaaagaa caattaacgc                              40


<210>  32
<211>  31
<212>  DNA
<213>  artificial sequence

<220>
<223>  Oligonucleotide

<400>  32
tatgttcatg cgatggcgca ccagctgggc g                                       31


<210>  33
<211>  20
<212>  DNA
<213>  artificial sequence

<220>
<223>  Oligonucleotide

<400>  33
cgacaattgg attcaccacg                                                    20


<210>  34
<211>  21
<212>  DNA
<213>  artificial sequence

<220>
<223>  Oligonucleotide

<400>  34
gcggtattga aaggttggtg c                                                  21


<210>  35
<211>  21
<212>  DNA
<213>  artificial sequence

<220>
<223>  Oligonucleotide

<400>  35
ccgtgagaag aatcgcgatc g                                                  21


<210>  36
<211>  21
<212>  DNA
<213>  artificial sequence

<220>
<223>  Oligonucleotide

<400>  36
gcgtagtcgt gtaagtatcg c                                                  21

<210>  37
<211>  20
<212>  DNA
<213>  artificial sequence

<220>
<223>  Oligonucleotide

<400>  37
cggtggcatt tggtcgtgac                                                    20


<210>  38
<211>  20
<212>  DNA
<213>  artificial sequence

<220>
<223>  Oligonucleotide

<400>  38
gctgtttcaa catcgatcac                                                    20


<210>  39
<211>  20
<212>  DNA
<213>  artificial sequence

<220>
<223>  Oligonucleotide

<400>  39
gatggcagat gacagtacgc                                                    20


<210>  40
<211>  22
<212>  DNA
<213>  artificial sequence

<220>
<223>  Oligonucleotide

<400>  40
ggagtcgatt atggaagagg cg                                                 22


<210>  41
<211>  29
<212>  DNA
<213>  artificial sequence

<220>
<223>  Oligonucleotide

<400>  41
gccacagccg gaatcatact tggtttggg                                          29


<210>  42
<211>  22
<212>  DNA
<213>  artificial sequence

<220>
<223>  Oligonucleotide

<400>  42
cagcgtaaag tcgtgcggcc ag                                                22


<210>  43
<211>  83
<212>  DNA
<213>  artificial sequence

<220>
<223>  Oligonucleotide

<400>  43
ccaatgcatg agctgttgac aattaatcat ccggctcgta taatgtgtgg tctagatcac        60

acaggaaaca gaccatgggc tag                                               83


<210>  44
<211>  25
<212>  DNA
<213>  artificial sequence

<220>
<223>  Oligonucleotide

<400>  44
gcggtatcat caacaggctt acccg                                             25


<210>  45
<211>  105
<212>  DNA
<213>  artificial sequence

<220>
<223>  Oligonucleotide

<400>  45
gttaactacg gatgtacatt atggaactga cgactcgcac tttacctgcg cggaaacata        60

ttgcgctggt ggcacacgat caggcctggt gatgatggcg ggatc                      105


<210>  46
<211>  120
<212>  DNA
<213>  artificial sequence

<220>
<223>  Oligonucleotide

<400>  46
gggaaattaa gttaccggta gtgcctgttg catacagtac gtgttgttcc agtaacggtt        60

gatgccgttc cacccagctc atcagcatct gtttgcattc agaagaactc gtcaagaagg       120


<210>  47
<211>  65
<212>  DNA
<213>  artificial sequence

<220>
<223>  Oligonucleotide

<400>  47
tctagagtag tcactactac gcgacctagg aggttcttta tggctatccc tgcatttggt        60

ttagg                                                                 65


<210>  48
<211>  35
<212>  DNA
<213>  artificial sequence

<220>
<223>  Oligonucleotide

<400>  48
tacgtagcta gcttaatccc attcaggagc cagac                                35


<210>  49
<211>  36
<212>  DNA
<213>  artificial sequence

<220>
<223>  Oligonucleotide

<400>  49
tcccccggga attaaccctc actaaagggc ggccgc                               36


<210>  50
<211>  35
<212>  DNA
<213>  artificial sequence

<220>
<223>  Oligonucleotide

<400>  50
tcccccgggt aatacgactc actatagggc tcgag                                35


<210>  51
<211>  47
<212>  DNA
<213>  artificial sequence

<220>
<223>  Oligonucleotide

<400>  51
tcctacgtat cacacaggaa acagaccatg gctatccctg catttgg                   47


<210>  52
<211>  30
<212>  DNA
<213>  artificial sequence

<220>
<223>  Oligonucleotide

<400>  52
ggaagatctt taatcccatt caggagccag                                      30


<210>  53
<211>  20
<212>  DNA
<213>  artificial sequence

<220>

<223>  Oligonucleotide

<400>  53
cccagctcat caaccaggtc                                                   20


<210>  54
<211>  19
<212>  DNA
<213>  artificial sequence

<220>
<223>  Oligonucleotide

<400>  54
cagcagaacc caggccagc                                                    19